# EUROPEAN PATENT APPLICATION

(11) **EP 2 832 863 A1**
(43) Date of publication of application: **04.02.2015**
(21) Application number: 13768660.6
(22) Date of filing: 28.03.2013
(51) Int. Cl.: C12Q 1/68, C12M 1/00, C12N 15/09

(54) **MICROARRAY FOR DETECTION OF MUTATIONS IN -GLOBIN GENES AND DETECTION METHOD THEREOF**

(30) Priority: 29.03.2012 JP 2012077394
(71) Applicant: Mitsubishi Rayon Co., Ltd., Tokyo 100-8253 (JP)
(72) Inventor: TOGAWA, Naoyuki, Yokohama-shi Kanagawa 230-0053 (JP)
(74) Representative: Reitstötter Kinzebach
(86) International application number: PCT/JP2013/060261
(87) International publication number: WO 2013/147320

(57) **Abstract**

Provided is a microarray for detecting mutations in a *β*-globin gene, which is capable of detecting a large number of mutations (specimens) conveniently in a short time. A probe group containing genes having the sequences set forth in SEQ ID NOs:3, 4, 7, 8, 11, 12, 17, 18 and 25 to 66 for detecting mutations in a *β*-globin gene, a microarray having the probe group immobilized thereon, a method for detecting mutations in a *β*-globin gene using the microarray, and a kit for *β*-globin gene mutation detection using the microarray and primers are provided.

## Description

### TECHNICAL FIELD

This invention relates to a probe group for detecting mutations in *β*-globin gene, a microarray having the same, and method for detecting a mutation in *β*-globin gene using the same.

### BACKGROUND ART

The human genome is composed of about three billion genetic codes (bases), but it has been found that there exist many differences in the genetic codes (base sequences) between individuals. Currently, among these differences of base sequences, the concern over the studies of single nucleotide polymorphism (SNP) has risen.

Single nucleotide polymorphism (SNP) means that only a single base in the base sequence of DNA is different, and this corresponds to a minimum unit of human personality, such as whether one can hold one's drink or not, or whether one is sensitive to a medicine or not. It has been suggested that among the three billion base pairs of the human genome, there are about 3,000,000 (proportion corresponding to one out of 500 to 1000 base pairs) to 10,000,000 single nucleotide polymorphisms, and these prevent production of a specific protein, or cause production of a protein that is different from that of other people, thereby bringing about differences such as differences among individuals (physical constitution) or differences among races. It is believed that the study of individual differences in the genes of the human being enables order-made medicine, by which single nucleotide polymorphisms are analyzed to investigate the susceptibility to a disease or the responsiveness to a drug, and a drug that would cause less adverse side effects in an individual person is administered to the relevant person. Thus, research on the analysis of single nucleotide polymorphism (SNP) is underway.

The reason why single nucleotide polymorphism (SNP) is attracting attention is that an analysis of a large number of SNP's has been made possible by a progress in the nucleic acid analysis technologies, and the correlation between diseases and SNP has been revealed. The correlation with SNP is being disclosed in a wide variety of domains such as disease-related genes, analysis of individual differences in drug metabolism, and chronic diseases. It is also expected that the correlation of these factors with SNP will be further disclosed in the future.

The nucleic acid analysis technologies handle a very large number of samples, and include an enormous number of operations; however, the technology is complicated and time-consuming, and generally, high accuracy is required. Among the nucleic acid analysis technologies, it has been known that a DNA chip for SNP detection (a DNA chip is also referred to as a DNA microarray; hereinafter, unless particularly stated otherwise, the terms will be considered to have the same meaning) is effective as a means for detecting a large number of genetic variations rapidly with high accuracy.

A DNA chip is a chip on which nucleic acid probes (probes) are respectively immobilized in defined compartments of a carrier, and usually, a single-stranded DNA or oligonucleotide molecule having a base sequence complementary to the nucleic acid fragment to be detected is used as the nucleic acid probe.

In a DNA chip for SNP detection, complementary strands of the nucleic acid fragments corresponding to the mutation sites of the test nucleic acid are immobilized as the nucleic probes. The test object sites for mutation usually include one normal type and plural variants, and nucleic acid probes matching any of those are arranged within a plot. Regarding the sample to be tested, a specimen liquid in which only a nucleic acid fragment corresponding to a mutated test object site has been amplified by a nucleic acid amplification method represented by a PCR method is used.

This specimen liquid is brought into contact with the surface of the DNA chip for SNP detection, on which the nucleic acid probes are immobilized, and the specimen nucleic acid fragment and the nucleic acid probe are hybridized. Then, the binding caused by this hybridization is detected as an optical or electrochemical signal, and thereby, the specimen nucleic acid fragments bound to the nucleic acid probes may be classified and quantitatively determined.

Here, when the combination of the nucleic acid probe and the specimen is a perfect match such as the combination of a wild type probe and a wild type specimen, or the combination of a variant probe and a variant specimen corresponding thereto, the hybridization forms a complete and strong bonding. On the contrary, when the combination of the nucleic acid probe and the specimen is a mismatch such as the combination of a wild type probe and a variant specimen, or the combination of a variant probe and a wild type specimen, since a site incapable of hydrogen bonding is inevitably accompanied, the hybridization is incomplete and forms a weak bonding.

Generally, hybridization is carried out under highly stringent conditions that are achieved by various combinations of temperature, a salt, a detergent, a solvent, a chaotropic agent, and a denaturant in order to maintain high specificity, and the difference in signal intensity originating from the difference in bonding force of hybridization between a perfect match and a mismatch is detected. Thereby, the genotype in the specimen may be identified and determined.

Meanwhile, hemoglobin is an iron-containing complex allosteric protein that transports oxygen from the lungs to the cells, and transports carbon dioxide from the cells to the lungs. Hemoglobin A is a key mature hemoglobin protein, and includes four polypeptide chains (two α-globin chains and two *β*-globin chains).

Many human diseases are considered to be caused by genetic variations that affect one or more genes encoding the hemoglobin polypeptide chains. Such diseases include sickle cell anemia, and are caused by point mutations in the *β*-chain of hemoglobin. Further, *β-*thalassemia symptoms relate to a blood-related disease caused by genetic variation that is significantly expressed in the phenotype by insufficient synthesis of one form of the globin chains, and cause excessive synthesis of the globin chains of the other form (see, for example, Non-Patent Document 1).

On the other hand, the recent development of the molecular biological techniques enables studies on the gene abnormalities causing or associated with the state and symptoms of particular human illness. The polymerase chain reaction (PCR) and many techniques derived therefrom by modifications serve as particularly useful tools for studying genetic abnormalities in the state and symptoms of illness (see, for example, Non-Patent Documents 2 and 3).

The use of the PCR method amplifies a particular target DNA or a portion of the DNA, and facilitates further characterizations of the amplified portion. Such further characterizations include gel electrophoresis for the determination of size, determination of the nucleotide sequence, studies on hybridization using particular probes, and so on (see, for example, Non-Patent Document 4).

In recent years, extensive studies have been carried out on the causal relationship between the genotype (that is, genetic polymorphism) such as SNPs (single nucleotide polymorphisms) and diseases and so on, and thus, decisions have been made on whether or not genetic abnormalities exist in the genome of a specific individual.

Regarding a method for determining (detecting) single base mutations such as SNPs, or a genetic variation with a number of bases of 2 or higher, first, a PCR-SSP method is available. Since this technique involves synthesis of primers specific to the base sequence of a mutated gene to perform PCR, several ten primer pairs are needed in order to discriminate several tens of genetic variations.

This method is a convenient method with short analysis time, but since scrupulous attention and knowledge, and time are required when such primers are designed, there are limitations in the analysis of a large amount of specimens. In addition, as there are more sites for which it is desired to detect mutation, the number of PCR-SSP also increases; therefore, there is a defect that it is difficult to handle a large number of specimens at a single time.

As a second method, a restriction enzyme fragment length polymorphism method (PCR-RFLP method) may be considered. Primers are designed in a consensus sequence site, and polymorphisms are included within the PCR product. After the amplification, the amplification product is cut using various restriction enzymes, and mutations of the gene sequence are classified based on the size of the DNA fragments.

This method allows easy determination of the results, and the method is also simple; however, when the sites capable of recognizing the restriction enzyme are limited, discrimination is made difficult. Further, since polyacrylamide gel should be used for the separation of the specimen, it is difficult to classify a large amount of specimen or several tens of mutations simultaneously. It has also been reported that generally, when a whole blood specimen is directly subjected to PCR amplification and then fragmentized with restriction enzymes, whole blood-derived proteins remain in the amplified specimen, and cutting by restriction enzymes is achieved imperfectly. That is, since proteins bound to a DNA are not separated from the DNA, restriction enzymes cannot bind to the DNA, the cleavage reaction cannot proceed normally, and there is a need for a DNA extraction operation.

A PCR-SSCP method is available as a third method. This method is a method of modifying PCR products into single-stranded DNAs (ssDNAs) by adding a modifying agent such as formamide, and then performing electrophoresis using a non-modified polyacrylamide gel. In regard to the electrophoresis, the ssDNAs respectively assume characteristic structures based on their base sequences, and exhibit intrinsic migration velocities in the electrophoresis, thereby forming bands of respectively different types.

This method is a method of classifying mutations in a base sequence by utilizing the property that ssDNAs exhibit intrinsic migration velocities based on the base sequences; however, a complicated technology with a high degree of difficulty is required, and the analysis of the results also requires experience and knowledge.

As a fourth method, a PCR-SSO (sequence specific oligonucleotide) method may be considered. PCR-SSO is a method of hybridizing synthetic probes for a normal site and a mutation site with PCR products that have been dotted on a filter (a microplate may also be used), and thereby detecting the presence or absence of mutations. On the contrary, there is also a reverse dot blotting method of dotting probes, and hybridizing the PCR products. To compare this method with the antigen-antibody reaction, this is a method in which a DNA serves as an antigen, and an antibody specific to a mutated site and an antibody specific to a normal site are caused to act as the antibodies has been bound to the DNA. Traditionally, radioactive isotopes have been used for the detection in this method, but due to a restriction on the used facilities and so on, detection is now achieved by chemiluminescence, color development method, or the like.

Although this method is simple, it is necessary to secure a significant amount of a sample, or else, it is necessary to adopt a technique for increasing the sensitivity (see, for example, Non-Patent Documents 5 and 6, and Patent Document 1).

As a fifth method, there is a direct base sequence determination method, which is a method of directly determining a base sequence by using a PCR-amplified DNA strand as a template, without performing subcloning into a vector or the like.

This method performs secondary PCR, which is called asymmetric PCR, of a PCR-amplified DNA strand to amplify a single-stranded DNA, and thereby determines the base sequence generally using a dideoxy method. This secondary PCR performs PCR using one member of a primer pair in a limited amount (usually 1:10 to 1:100), and thereby a single-stranded DNA is amplified. Recently, a cycle sequencing method has been applied so that a sequencing reaction can now be carried out more simply. However, since the price of the kit is very expensive, highly expensive apparatuses are required, and the experimental procedure is also complicated, it is cost-consuming in order to analyze a large amount of a specimen.
Patent Document 1: JP 5-184398 A
Non-Patent Document 1: Weatherall et al., The Thalassaemia Syndromes, 3rd Edition, Oxford Blackwell Scientific, 1981
Non-Patent Document 2: Erlich et al., Current Communications in Molecular Biology: Polymerase Chain Reaction, Cold Spring Harbor: Cold Spring Harbor Press (1989)
Non-Patent Document 3: Innis et al., PCR Protocols: A Guide to Methods and Applications. San Diego: Academic Press (1990)
Non-Patent Document 4: Sambrook et al., Molecular Cloning: A Laboratory Manual, 2nd Edition, Cold Spring Harbor: Cold Spring Harbor: Cold Spring Harbor Press (1989)
Non-Patent Document 5: Am. J. Hum. Genet., 43:095-100, 1988
Non-Patent Document 6: Blood, Vol. 81, No. 1 (January 1), 1993: pp. 239-242

### DISCLOSURE OF THE INVENTION

As described above, various detection methods have been used to analyze and detect mutations of genes, but a common drawback is that a very long time and enormous efforts are required to simultaneously detect a large number of mutations, and it is even more difficult on analyzing a large amount of a specimen.

Hence, a primary object of this invention is to provide a microarray for detecting mutations in a *β*-globin gene, which can detect a large number of mutations (specimen) simply and conveniently in a short time.

In view of the problems of the related art, the inventors of this invention conducted thorough investigations and thereby found that the object described above can be achieved by using plural kinds of probes having particular sequences, thus completing this invention.

That is, this invention relates to a probe group for detecting mutations in a *β*-globin gene that contains genes having the sequences set forth in SEQ ID NOs:3, 4, 7, 8, 11, 12, 17, 18, and SEQ ID NOs:25 to 66, a microarray having the probe group immobilized thereon, and a method and a kit for detecting mutations using the microarray.

According to this invention, since a hybridization solution can be mixed in so as to be brought directly into contact with and react with the microarray without purifying the PCR product, even in a case where a large amount of a specimen is used, the treatment may be carried out in short time. Further, since 25 sites of mutation in a *β*-globin gene may be detected all at once, this invention is good in practical usability and usefulness.

### BRIEF DESCRIPTION OF DRAWINGS

Fig. 1 shows diagrams illustrating the correction method of the invention.
Fig. 2 shows diagrams illustrating the correction method of the invention.
Fig. 3 shows diagrams illustrating the correction method of the invention.
Fig. 4 shows diagrams illustrating the correction method of the invention.
Fig. 5 shows diagrams produced by plotting the results of the hybridization of a first control nucleic acid performed plural times, in a fluorescence coordinate system representing the signal intensities of the first and the second probes for polymorphism detection, and presenting representative straight lines thereof.
Fig. 6 shows diagrams produced, in addition to Fig. 5, by plotting the results of the hybridization of a second control nucleic acid performed plural times, and presenting representative straight lines thereof.
Fig. 7 shows graphs for the correction values C and C₂.
Fig. 8 shows the probe performance data obtained before and after making corrections using the correction values C and C₂, and the angle of error.
Fig. 9 shows diagrams illustrating the data obtained before correction and after correction, from 25 kinds of plasmid-derived samples.
Fig. 10 is a graph showing the results obtained by superimposing the data of Table 9 on the graph of Fig. 9 for the data obtained after correction.

### BEST MODES FOR CARRYING OUT THIS INVENTION

Hereinafter, this invention will be described in detail. The following embodiment is an exemplary embodiment for explaining this invention, and is not intended to limit this invention. This invention may be carried out in various forms as long as the gist is maintained.

Meanwhile, all publications, patent applications, and patent documents other than patent publications mentioned in this specification are incorporated herein by reference. Also, the present specification includes the subject matters described in the specification and the drawings of Japanese Patent Application No. 2012-077394 filed on March 29, 2012, from which the present application claims priority.

Hereinafter, this invention will be described in detail. The following embodiment is an exemplary embodiment for explaining this invention, and is not intended to limit this invention. This invention may be carried out in various forms as long as the gist is maintained.

Further, unless particularly stated otherwise, an amino acid sequence is defined to have the amino terminus at the left end and the carboxyl terminus at the right end, and a base sequence is defined to have the 5'-terminus at the left end and the 3'-terminus at the right end.

### 1. Probe for polymorphism detection

A microarray is generally used for the detection of a polymorphism, but in order to perform detection with high sensitivity, there is a demand for a high performance probe which does not easily undergo non-specific hybridization. The performance of a probe generally depends on the Tm value thereof (the higher the Tm value is, the more likely a non-specific hybridization occurs), the Tm value is determined by the sequence of the probe. Therefore, generally, the performance of the probe is constrained by the sequence of the peripheral region of the polymorphism to be detected.

However, the inventors of this invention succeeded in enhancing the performance of a probe by regulating the Tm value thereof by applying modification to the sequence of the probe to the extent that the intrinsic performance of the probe is not impaired.

Therefore, this invention provides a probe as described below.

The probe is for detecting a polynucleotide sequence having one or plural polymorphisms, characterized by being hybridized to the relevant sequence, and satisfying at least arbitrary one of the following requirements:
1) containing one or more non-complementary bases with respect to each end or one end of the sequence,
2) that the portion thereof corresponding to the polymorphisms not targeted for detection among the plural polymorphisms contained in the sequence contains universal bases, and
3) that the polymorphism targeted for detection is located at a position six or less bases away from any one terminus of the probe.

According to this invention, the term "probe" means a compound capable of capturing a substance targeted for detection that is included in a specimen. Examples thereof include nucleic acids such as a deoxyribonucleic acid (DNA), a ribonucleic acid (RNA), and a peptide nucleic acid (PNA), which may be obtained from commercially available synthetic products such as DNAs synthesized in vitro by enzymes or the like, and chemically synthesized oligonucleotides, or from live cells. A DNA fragment having been chemically modified or cleaved by a restriction enzyme may also be used.

The length of a probe is generally about 10 bp to 100 bp, preferably 10 bp to 80 bp, more preferably 10 to 50 bp, even more preferably 10 bp to 35 bp, and most preferably 12 bp to 28 bp.

Further, the "polynucleotide sequence having one or more polymorphisms", which is the object of detection, means a polynucleotide included in a specimen, having one or more, two or more, or three or more polymorphisms in the base sequence.

The polynucleotide as an object of detection is primarily derived from a human specimen, but as long as the polynucleotide may be subjected to an amplification reaction, a polynucleotide of any biological species may be used.

The specimen may be any of cells, blood or body fluid derived from any tissue. Specific examples of the specimen include cells, blood and body fluid derived from various tissues such as brain, heart, lung, spleen, kidney, liver, pancreas, gall bladder, esophagus, stomach, intestines, urinary bladder, and skeletal muscles of human being. More specific examples include blood, cerebrospinal fluid, urine, sputum, pleural fluid, ascitic fluid, gastric juice, and bullous fluid.

The polynucleotide as the object of detection may be purified before being supplied to the microarray. In regard to the method for purifying a polynucleotide, e.g., various technologies as described in Maniatis, et al. ("Molecular Cloning: A Laboratory Manual," Cold Spring Harbor, NY, pp. 280, 281, 1982) may be employed.

Feature 1) is that the probe contains one or more non-complementary bases with respect to each end or one end of the polynucleotide sequence containing polymorphisms as the object of detection

In general, a "GC-rich" polynucleotide having large contents of guanine (G) and cytosine (C) has a high Tm value and is likely to undergo non-specific hybridization.

Thus, the inventors of this invention found that in a case where the polynucleotide used as a probe contains a GC-rich region, a mismatch is caused between the probe and the polynucleotide as the object of detection by incorporating a GC-rich sequence and non-complementary bases at both ends of the same region, and thereby the Tm value of the probe may be decreased.

A "non-complementary base" means any base causing a mismatch with a corresponding base on the polynucleotide sequence as the object of detection. For example, when the corresponding base on the polynucleotide sequence as the object of detection is "C", the non-complementary base may be any of "A", "T" and "C". When the corresponding base on the polynucleotide sequence as the object of detection is "G", the non-complementary base may be any of "A", "T" and "G". When the corresponding base on the polynucleotide sequence as the object of detection is "A", the non-complementary base may be any of "A", "C" and "G". Furthermore, when the corresponding base on the polynucleotide sequence as the object of detection is "T", the non-complementary base may be any of "T", "C" and "G".

The number of non-complementary bases contained at the two ends may be different between the 5'-terminus and the 3'-terminus.

The "non-complementary base" according to this invention is incorporated into the two terminal sections of the polynucleotide sequence containing a polymorphism as the object of detection, or into the two terminal sections of the GC-rich sequence containing a polymorphism. For example, when the polynucleotide sequence containing a polymorphism as the object of detection is "GGCGCGGCGCGG" (the underlined part at the center is the polymorphism as the object of detection), the probe having the feature 1) may be "TGCGCGGCGCGA", may be "ATGCGCGGCGCGAA", or may be "ATGGCGCGGCGCGGAA".

Preferably, the "non-complementary base" includes one or more bases, two or more bases, or three or more bases, at either terminal section.

According to this invention, the GC-rich region means a sequence region in which the content of G and C contained in the entire base sequence is 50% or more, 55% or more, 60% or more, 61% or more, 62% or more, 63% or more, 64% or more, 65% or more, 70% or more, 75% or more, 80% or more, 85% or more, 90% or more, or 95% or more.

Feature 2) is that the portion of the probe corresponding to the polymorphisms not intended for detection among the plural polymorphisms contained in the polynucleotide sequence containing the polymorphisms as the objects of detection contains universal bases.

A probe having this feature is useful when a second polymorphism which is not an object of detection is contained, in addition to a first polymorphism which is the object of detection, in the polynucleotide sequence as the object of detection.

In this case, since the binding force between the probe and the polynucleotide as the object of detection is changed by the combination for the second polymorphism, in addition to the combination for the first polymorphism, the sensitivity of detection to the first polymorphism as the original object of detection is decreased.

When this polynucleotide sequence is used directly as the sequence of the probe, the polynucleotide as the object of detection in the first polymorphism portion matches the probe; however, there may be an occasion in which the polynucleotide and the probe do not match (mismatching) in the second polymorphism portion, and an occasion in which the polynucleotide as the object of detection and the probe do not match (mismatching) in the first polymorphism portion, while the polynucleotide and the probe match in the second polymorphism portion. In the latter case, despite the first polymorphism does not match, since the probe and the polynucleotide as the object of detection are hybridized with a binding force at the same level as that of the former case, positive signals similar to those of the former case are emitted.

Therefore, in regard to the above-described cases, the former (true positive) and the latter (false positive) cannot be distinguished.

Thus, in order to nullify the influence of the second polymorphism, the probe of this invention is characterized by containing universal bases in the portion corresponding to the second polymorphism.

According to this invention, a universal base means a base which does not form a base pair with any of naturally occurring nucleic acid bases, namely, adenine, guanine, thymine, cytosine, and uracil.

Examples of such a universal base include, but are not limited to, 5-nitroindole, 3-nitropyrrole, 7-azaindole, 6-methyl-7-azaindole, pyrrolepyridine, imidazopyridine, isocarbostyryl, propynyl-7-azaindole, propynylisocarbostyryl, and allenyl-7-azaindole.

Other examples of the universal base include any one or more of the following compounds, including propynyl derivatives thereof:
8-aza-7-deaza-2'-deoxyguanosine, 8-aza-7-deaza-2'-dioxyadenosine, 2'-deoxycytidine, 2'-deoxyuridine, 2'-deoxyadenosine, 2'-deoxyguanosine, and pyrrolo[2,3-d]pyrimidine nucleotide.

Furthermore, the universal base may be formed from any of the following compounds, including derivatives thereof:
Deoxyinosine (e.g., 2'-deoxyinosine), 7-deaza-2'-deoxyinosine, 2'-aza-2'-deoxy-inosine, 3'-nitroazole, 4'-nitroindole, 5'-nitroindole, 6'-nitroindole, 4-nitrobenzimidazole, nitroindazole (e.g., 5'-nitroindazole), 4-aminobenzimidazole, imidazo-4,5-dicarboxamide, 3'-nitroimidazole, imidazole-4-carboxamide, 3-(4-nitroazol-1-yl)-1,2-propanediol, and 8-aza-7-deazaadenine (pyrazolo[3,4-d]pyrimidine-4-amine).

In another example, regarding the universal nucleic acid base, a universal nucleic acid base may be formed by combining a 3-methyl-7-propynylisocarbostyryl group, a 3-methylisocarbostyryl group, a 5-methylisocarbostyryl group, an isocarbostyryl group, a phenyl group or a pyrenyl group, with ribose or deoxyribose.

Feature 3) is that the polymorphism intended for detection is located at a position six or fewer bases away from any one terminus of the probe.

Furthermore, the probe of this invention may also be designed in a manner such that the polymorphism intended for detection is located at a position six or less bases away from any one terminus (5'-terminus or 3'-terminus) of the probe.

A probe designed as such is useful in view of the following point.

Generally, when a probe for detecting a single nucleotide polymorphism is designed, the probe is designed to have a base length that is approximately equal on both the 5'-terminus side and the 3'-terminus side while centering the position of the polymorphism intended for detection. However, if the 5'-terminus side or the 3'-terminus side at the site of the polymorphism intended for detection is an extremely GC-rich region or an extremely AT-rich region, binding to the probe may occur, or may not occur, at the position of the polymorphism intended for detection, regardless of being a match or a mismatch.

Thus, this invention is characterized by using a probe in which the position of the polymorphism intended for detection is set to a position six or less bases away from a terminus, and thus a GC-rich region or an AT-rich region is avoided. In a case in which the specificity of the probe cannot be enhanced by employing this feature only, the feature 1) may be employed in combination.

There are no particular limitations on the gene that serves as a basis of the polynucleotide of this invention, and examples include G6PD gene, RAB27A gene, CHS1 gene, MTHFR gene, HMGCL gene, SLC2A1 gene, and H6PD gene. In addition, to obtain information individually, access may be made to the OMIM Database (http://www.ncbi.nlm.nih.gov/omim), where the information on a disease, and causes thereof or genes that serve as risk factors may be obtained.

According to an embodiment, the polynucleotide of this invention is prepared from the human *β*-globin gene.

According to this invention, the polynucleotide sequence having the polymorphism intended for detection has a sum of the contents of guanine and cytosine of 63% or more (GC-rich), and has nucleotide sequences represented by the 99^{th} to 117^{th} nucleotides, the 127^{th} to 142^{nd} nucleotides and the 1402^{nd} to 1416^{th} nucleotides of the human *β*-globin gene.

Alternatively, the polynucleotide sequence having the polymorphism intended for detection has a sum of the contents of guanine and cytosine of 45% or less (GC-poor), and has nucleotide sequences represented by the 1378^{th} to 1399^{th} nucleotides of the human *β*-globin gene.

The above-described region is a GC-rich region or an AT-rich region, and provides a probe capable of detecting a polymorphism in this site.

More specifically, the probe of this invention is a probe specialized by a GC-rich region, and has a sequence set forth in SEQ ID NO:3, 4, 7, 8, 17 or 18.

On the other hand, the probe of this invention is a probe specialized by a GC-poor region, and has a sequence set forth in SEQ ID NO:11 or 12.

Furthermore, this invention provides a microarray having at least one of sequences set forth in SEQ ID NOs:3, 4, 7, 8, 11, 12, 17 and 18.

### 2. Probe group

According to this invention, sequences set forth in SEQ ID NOs:3, 4, 7, 8, 11, 12, 17 and 18 and SEQ ID NOs:25 to 66 are used as probes (probe group of this invention), and if necessary, genes other than the probe group of this invention may also be used as probes.

### 3. Microarray

### (1) Support

In order to actually put the above probe group to use, it is necessary to immobilize the probes to a support. There are no limitations on the kind of the support for immobilization, and any support that does not allow a probe to be eluted (released) into the reaction liquid at the time of a hybridization reaction, and enables characterization of which probe has reacted after the reaction, may be used.

Examples of the support include a filter, beads, a gel, a chip, a slide glass, a multi-well plate, a membrane, and an optical fiber. More specific examples include a Western Blotting filter paper, a nylon membrane, a membrane made of polyvinylidene fluoride, a nitrocellulose membrane (Pierce Biotechnology, Inc.), affinity beads (Sumitomo Bakelite Co., Ltd.), MicroPlex (registered trademark) Microspheres, xMAP Multi Analyte LumAvidin Microspheres (Luminex Corp.), Dynabeads (Veritas Corp.), a 96-well plate kit for DNA immobilization (Funakoshi Co., Ltd.), a substrate for DNA immobilization (Sumitomo Bakelite Co., Ltd.), a coated slide glass for microarray (Matsunami Glass Industry, Ltd.), a hydrogel slide (PerkinElmer, Inc.), and Sentrix (registered trademark) Array Matrix (Illumina, Inc.).

### (2) Immobilization

Immobilization of a probe may be carried out, in the case of using a filter or a membrane, etc., by directly spotting an unmodified probe, and irradiating the probe with a UV lamp or the like. Furthermore, in the case of using beads, a chip, a slide glass, a multi-well plate, a membrane, an optical fiber and so on, which have their surfaces chemically activated, it is preferred to use a probe having a terminus that may form chemically covalent bonding. More specifically, a probe having an amino group or the like introduced to the 5'-terminus or the 3'-terminus is used. Further, in the case of immobilizing a probe onto a gel or the like, a probe having an unsaturated functional group that is capable of copolymerization reaction is used. When the probe has this introduced group, the probe is immobilized to the network structure of the gel by a copolymerization reaction with a substituted (meth)acrylamide derivative or an agarose derivative, and a crosslinking agent. In regard to the method of introducing an unsaturated functional group into the terminus of a nucleic acid strand, for example, the known method as described in WO 02/062817 may be used.

In this invention, it is preferred to immobilize the probe onto a gel or within a gel. This is because when the probe is immobilized within a gel, the amount of the probe may be increased to increase the detection sensitivity of the microarray. Furthermore, in this invention, it is preferred to maintain the gel inside through-holes, and to use a through-hole type microarray having a plural number of the relevant through-holes.

A through-hole type microarray may be obtained by forming through-holes on a foil plate, but a microarray is preferably obtained by retaining gel carriers having probes immobilized therein, in the hollow sections of tubular bodies such as hollow fibers such that different kinds of the gel carriers are retained in different tubular bodies, gathering and fixing all the tubular bodies such as hollow fibers, and then repeatedly cutting the tubular bodies along the longitudinal direction of the fibers. This is because microarrays of stable quality may be produced in large quantities. In this manner, a microarray in which respective probes are immobilized within various through-holes in an independent manner (a state in which a probe of one kind is immobilized within one through-hole), may be obtained.

Hereinafter, an embodiment of the method for producing a through-hole type microarray is explained. The relevant microarray may be produced with the steps i) to iv) described below.

Step i) is to arrange plural lines of hollow fibers three-dimensionally such that the fiber axes of the various hollow fibers are in the same direction, fix the arrangement with a resin, and thereby produce a hollow fiber bundle

The method for forming through-holes is not particularly limited, and for example, a method of producing an arranged body in which hollow fibers are arranged in the same axial direction and then fastening the arranged body with a resin, as described in JP 2001-133453 A, may be utilized. Regarding the hollow fibers, various materials may be used, but an organic material is preferred.

Examples of a hollow fiber formed from an organic material include: polyamide-based hollow fibers of nylon 6, nylon 66, aromatic polyamide, and so on; polyester-based hollow fibers of polyethylene terephthalate, polybutylene terephthalate, polylactic acid, polyglycolic acid, polycarbonate, and so on; acrylic hollow fibers of polyacrylonitrile and so on; polyolefin-based hollow fibers of polyethylene, polypropylene, and so on; polymethacrylate-based hollow fibers of polymethyl methacrylate and so on; polyvinyl alcohol-based hollow fibers; polyvinylidene chloride-based hollow fibers; polyvinyl chloride-based hollow fibers; polyurethane-based hollow fibers; phenolic hollow fibers; fluorine-based hollow fibers formed from polyvinylidene fluoride, polytetrafluoroethylene, and so on; and polyalkylene para-oxybenzoate-based hollow fibers. The hollow fibers may be porous, and may be obtained by combining a melt spinning method or a solution spinning method with known porosification technologies such as a stretching method, a microphase separation method, and an extraction method. The porosity is not particularly limited, but in view of increasing the density of the probes to be immobilized per unit length of the fiber material, a higher porosity is preferred as the specific surface area increases. The inner diameter of the hollow fiber may be arbitrarily set. The inner diameter may be adjusted preferably to 10 µm to 2000 µm, and more preferably 150 µm to 1000 µm.

The method for producing the relevant hollow fiber is not limited, and the hollow fiber may be produced by a known method such as the one described in JP 11-108928 A. For example, a melt spinning method is preferred, and regarding the nozzle, a horseshoe-shaped nozzle, a C-shaped nozzle or a double pipe nozzle, etc., may be used. According to this invention, it is preferred to use a double pipe nozzle from the viewpoint that a continuous and uniform hollow section may be formed.

Further, if necessary, a hollow fiber in which a black pigment such as carbon black has been included in an appropriate amount, may also be used. When the hollow fiber contains a black pigment, optical noises originating from foreign materials such as impurities may be reduced at the time of detection, or the strength of the resin may be increased. The content of the pigment is not limited, and the content may be appropriately selected according to the size of the hollow fiber, the purpose of use of the microarray, and so on. For example, the content may be adjusted to 0.1% to 10% by mass, preferably 0.5% to 5% by mass, and more preferably 1% to 3% by mass.

Production of a block body may be carried out using a method of fixing the block body with a resin such as an adhesive so that the arrangement of the arranged body would not be disrupted. For example, there is a method of arranging plural lines of hollow fibers in parallel at a predetermined interval on a sheet-like object such as an adhesive sheet, fabricating the assembly into a sheet form, and then winding this sheet into a helical form (see JP 11-108928 A). Another method may include superimposing two sheets of porous plates each having plural holes provided at a predetermined interval, such that the respective hole areas of the plates would coincide, passing hollow fibers through those hole areas, opening a gap between the two sheets of porous plates, filling a curable resin raw material around the hollow fibers between the two sheets of porous plates, and curing the resin raw material (JP 2001-133453 A).

The curable resin raw material is preferably formed from an organic material such as a polyurethane resin or an epoxy resin. Specifically, the curable resin raw material is preferably formed from one or more kinds of materials consisting of organic polymers and so on. Examples of the organic polymer include: rubber materials, such as polyurethane, a silicone resin, and an epoxy resin; polyamide-based resins, such as nylon 6, nylon 66, and an aromatic polyamide; polyester-based resins, such as polyethylene terephthalate, polybutylene terephthalate, polylactic acid, polyglycolic acid, and polycarbonate; acrylic resins, such as polyacrylonitrile; polyolefin-based resins, such as polyethylene and polypropylene; polymethacrylate-based resins, such as polymethyl methacrylate; polyvinyl alcohol-based resins; polyvinylidene chloride-based resins; polyvinyl chloride-based resins; phenolic resins, fluorine-based resins, such as polyvinylidene fluoride and polytetrafluoroethylene; and polyalkylene para-oxybenzoate-based resins. In the organic polymer, a black pigment such as carbon black may be incorporated in an appropriate amount. When a black pigment is added, optical noises originating from foreign materials such as impurities may be reduced at the time of detection, or the strength of the resin may be increased. The content of the pigment is not limited, and the content may be appropriately selected according to the size of the hollow fiber, the purpose of use of the microarray, and so on. For example, the content may be adjusted to 0.1% to 10% by mass, preferably 0.5% to 5% by mass, and more preferably 1% to 3% by mass.

The number of the hollow fibers that are arranged in this invention, that is, the number of spots, is not limited and may be appropriately selected according to the intended experiment or the like. Therefore, the distance between the hollow fibers may also be appropriately selected according to the area of the microarray, and the number of the hollow fibers to be arranged, etc.

Step ii) is to introduce a gel precursor solution containing a probe group into the hollow section of each hollow fiber of the hollow fiber bundle

The kind of the gel material that is filled in the hollow fibers is not particularly limited, and polysaccharides such as agarose and sodium alginate, and proteins such as gelatin and polylysine may be used, as long as the gel material is a gel material obtainable from natural products. Regarding synthetic polymers, for example, a gel that is obtained by allowing a polymer having a reactive functional group such as polyacryloylsuccinimide to react with a crosslinking agent having reactivity with the polymer may be utilized. In addition, preferred examples also include synthetic polymer gels obtained by using polymerizable monomers such as acrylamide, N,N-dimethylacrylamide, N-isopropylacrylamide, N-acryloylaminoethoxyethanol, N-acryloylaminopropanol, N-methylolacrylamide, N-vinylpyrrolidone, hydroxyethyl methacrylate, (meth)acrylic acid and allyl dextrin as monomers, and copolymerizing the monomers with a polyfunctional monomer such as methylenebis(meth)acrylamide or polyethylene glycol di(meth)acrylate.

The concentration of the gel used in the microarray of this invention is not particularly limited, and the concentration may be appropriately selected according to the length or amount of the probe used. For example, the concentration in terms of the concentration of the monomer component is preferably 2% to 10% by mass, more preferably 3% to 7% by mass, and even more preferably 3.5% to 5% by mass. The concentration is adjusted to 2% by mass or more because the probes may be securely immobilized so that the detection of the target substance may be carried out with high efficiency. Furthermore, the concentration is adjusted to 10% by mass or less because even though the concentration is made higher than that, it may be difficult to obtain a dramatically improved effect.

In the case of retaining a synthetic polymer gel in the microarray of through-hole substrates described above, the synthetic polymer gel may be retained by filling a gel precursor solution of the synthetic polymer in the above-described block, and then gelating the gel precursor solution within the block. Regarding the method of filling a gel precursor solution inside the through-holes of the block, for example, the solution may be introduced by suctioning the solution into a syringe having a fine needle, and inserting the needle into the hollow section of each hollow fiber. Furthermore, the hollow section of the fixed end of the hollow fiber bundle is sealed, and the hollow section of the other non-fixed end is left open. Next, a gel precursor solution containing a nucleic acid probe having a polymerization reaction point such as a methacryl group at a terminus is prepared, the gel precursor solution and the hollow fiber bundle are placed in a desiccator, subsequently the end of the hollow fiber bundle at which the hollow fibers are not fixed is immersed in this solution, the interior of the desiccators is brought to a state under reduced pressure, and then the pressure is returned to normal pressure. Thereby, this solution may be introduced into the hollow section of the hollow fibers through the ends of the hollow fibers immersed in the solution.

Step iii) is to cause the gel precursor solution that has been introduced into the hollow section of the hollow fiber bundle to react, and thereby maintain a gel-like material containing probes in the hollow section of the hollow fibers

By polymerizing the gel precursor solution that has been introduced into the hollow section of the hollow fibers, a gel-like material containing probes is retained in the hollow section of the hollow fibers. The conditions for polymerization are not particularly limited, and may be appropriately selected depending on the kind of the gel precursor used, etc. For example, an acrylamide-based monomer may be polymerized using a radical initiator, and preferably, an acrylamide-based monomer may be polymerized by a thermal polymerization reaction using an azo-based initiator.

The kind and size of the probe are not limited, and may be appropriately selected according to the kind of the substance or compound that is the object of detection.

Step iv) is to cut the hollow fiber bundle in a direction perpendicular to the longitudinal direction of the fibers, and thereby slice the hollow fiber bundle

The method for cutting is not limited as long as slices may be obtained. For example, the cutting may be carried out using a microtome, or a laser, etc. The thickness of the slice thus obtained is not limited, and may be appropriately selected according to the purpose of the experiment, etc. For example, the thickness may be adjusted to 5 mm or less, and preferably to 0.1 mm to 1 mm.

### (3) Detection of mutation in β-globin gene

According to this invention, detecting mutations in the *β*-globin gene means characterizing the base site having a mutated portion in the *β*-globin gene sequence, and the sequence, and also means that it is determined which pair of alleles (diploid organism) has the mutation among plural specified alleles.

(a) First, it is desired to bring a specimen containing a human genome DNA into contact with a reaction solution containing a primer set for nucleic acid amplification, a nucleotide unit, and a DNA elongation enzyme.

### <Specimen (nucleic acid serving as template of nucleic acid amplification)>

A specimen refers to a nucleic acid containing the gene sequence targeted for detection in this invention, that is, the *β*-globin gene sequence. Any form of nucleic acid may be used, as long as it contains a fragment of the *β*-globin gene sequence and is capable of undergoing the amplification reaction described below.

The specimen is human-derived, and any material capable of an amplification reaction may be used. For the specimen, cells, blood or body fluid derived from any tissue may be used. Examples include cells, blood and body fluid derived from various tissues such as brain, heart, lung, spleen, kidney, liver, pancreas, gall bladder, esophagus, stomach, intestines, urinary bladder, and skeletal muscles. More specific examples include blood, cerebrospinal fluid, urine, sputum, pleural fluid, ascitic fluid, gastric juice, and bullous fluid.

Further, it is preferred to prepare and purify the specimen as a DNA-containing sample that may be used for the nucleic acid amplification as described below, before the relevant amplification is carried out. This preparation and purification may be carried out with a known nucleic acid extraction method, and for example, various technologies as described in Maniatis, et al. ("Molecular Cloning: A Laboratory Manual," Cold Spring Harbor, NY, pp. 280, 281, 1982) may be used.

### <Primer set for nucleic acid amplification and probe position>

This invention relates to a probe set for detecting mutations in the *β*-globin gene, and detects mutations in the *β*-globin gene sequence encoded on a complementary strand sequence ofNCBI Reference Sequence: NC_000011.9 (sequence length 135006516 bases).

Usually, it is preferred to have the site of mutation in the nucleic acid to be detected, from the viewpoint of detection. The primer pair may be designed to include any region, as long as an amplification product containing a mutation site to be detected is produced. For example, when it is intended to detect plural mutation sites that are contained in exons 1 and 2 all at once, nucleic acid amplification may be carried out using a forward primer on the upstream of exon 1 and a reverse primer on the downstream of exon 2, and the amplification products may be detected. Further, when it is intended to detect plural regions at the same time, plural primer pairs may be used. In this case, it is preferred to check whether or not non-specific nucleic acid fragments have been produced in the stage of performing amplification, at the stage of setting the conditions. Once the conditions are determined, detection may be carried out with high reproducibility under those conditions.

For the base sequence of the oligonucleotide that serves as a probe, the probe sequence is determined as included in the amplification product sequence (region sandwiched between the primers of the primer set). The length of the probe is usually set to about 15 to 35 nucleotides, and for one mutated region to be detected, usually a pair (two kinds, namely, a probe for wild type detection and a probe for mutant detection) or more pairs of probes are required.

For the purpose of facilitating the subsequent detection, the primer set to be used may have the termini labeled in advance with a fluorescent substance (Cy3, Cy5 or the like), or biotin, etc. There are no particular limitations on the method of labeling, and any method may be used as long as the method does not exhibit any phenomenon against the amplification reaction, such as marked inhibition of the reaction. After the reaction, color development may also be induced by further causing the reaction system to react with a complex with an enzyme, for example, streptavidin-alkaline phosphatase conjugate, and adding a substrate thereto.

### <Amplification reaction>

The nucleic acid (specimen) that serves as the template for nucleic acid amplification is used as a template, and a nucleic acid fragment of the region to be detected from the mutation site in the *β*-globin gene is amplified. Regarding the method for amplification of nucleic acid, various methods such as a PCR method, a LAMP method, an ICAN method, a TRC method, a NASBA method, and a PALSAR method may be used. Any method may be used for the nucleic acid amplification reaction as long as there is no particular problem in view of the detection of nucleic acid, but among these, a PCR method is preferred in view of convenience.

As a temperature controlling apparatus used in the nucleic acid amplification reaction, a commercially available thermal cycler may be used. For example, GeneAmp 9600 or GeneAmp 9700 (Life Technologies Japan, Ltd.), or T Professional Series (Biometra GmbH), may be used, but an apparatus of any type may be used as long as there is no problem with thermal conductivity and the shape of the lid.

### <Nucleotide unit used in amplification reaction>

An example of the nucleotide unit is deoxyribonucleotide triphosphate or the like, which is used in conventional amplification reactions. As for this, a derivative that facilitates detection later may be used as in the case of the primer set; however, it is preferred to use a nucleotide unit that does not inhibit the amplification reaction.

### <DNA elongation enzyme used in amplification reaction, and other enzymes>

Regarding DNA elongation enzymes, TaqDNA polymerase, TthDNA polymerase, PfuDNA polymerase and so on, which are DNA polymerases derived from heat resistant bacteria, may be used in the same manner as in the case of being used in a conventional PCR method.

Examples of an enzyme or a kit that may be used include Hot StarTaq DNA Polymerase (by Qiagen Corp.), PrimeStarMax DNA polymerase (Takara Bio, Inc.), SpeedSTAR HS DNA polymerase (Takara Bio, Inc.), KOD Plus Neo (Toyobo Co., Ltd.), KAPA2G FastHotStart PCR Kit (Nippon Genetics Co., Ltd.), and AmDirect kit (Shimadzu Corp.). In addition to these, any enzyme or kit capable of performing a nucleic acid amplification reaction directly from blood (body fluid or the like), is more preferred since the operation is made simple thereby.

Regarding the method of mixing the above constituent elements described, any mixing method may be used as long as the enzyme is not deactivated, and mixing is achieved without causing the liquid to foam and leak from the tube. Usually, all the above constituent elements are dispensed in a tube for PCR use having a size of about 0.2 mL, the mixture is mixed using a vortex mixer, and then the mixture may be lightly centrifuged (spin-down) in order to cause the solution adhering to the lid to fall off. Further, in a case of performing HotStart PCR, mixing is performed under the conditions in which the enzyme is not activated at the time of mixing.

Next, in step b), the reaction liquid obtained in the above step a) may be subjected to a nucleic acid amplification reaction.

In regard to the nucleic acid amplification reaction, for example, in the case of performing amplification by a PCR reaction, an enzyme which dissociates the nucleic acid that serves as a template is activated at 90°C to 98°C for about 5 min, subsequently a cycle of 30 sec at 94°C (dissociation of nucleic acid), 30 sec at 60°C (annealing of primers), and 30 sec at 72°C (elongation reaction from primers) is repeated 25 to 50 times, and thereby the nucleic acid may be amplified logarithmically. Further, in a case of performing a nucleic acid amplification reaction under an isothermal condition instead of a PCR reaction, amplified nucleic acid may be obtained by incubating the reaction system at a constant temperature at about 40°C to 65°C.

c) Subsequently, the nucleic acid fragment obtained in step b) is brought into contact with the microarray of this invention, and thereby the targeted nucleic acid in the specimen may be detected.

In step c), the nucleic acid amplification reaction liquid may be brought into contact with the microarray by directly adding a hybridization solution, without purifying the nucleic acid amplification reaction liquid. A hybridization solution is a solution enabling the amplified nucleic acid to undergo a hybridization reaction with the probe immobilized in the microarray.

More specifically, the hybridization solution is a solution obtained by mixing a solution mixed with a salt, such as a NaCl solution or a MgCl₂ solution, a SSC solution, and a surfactant such as SDS or Tween 20, into a buffer solution such as Tris/HCl buffer. In the case of performing a reaction with the probe used in this invention, it is preferred to use TNT buffer (mixed solution of a Tris/HCl buffer solution, a NaCl solution, and a Tween solution), in which crystals of a surfactant, such as SDS, are not precipitated at the time of cooling.

The concentration of Tris/HCl or NaCl is preferably 0.06 to 0.48 M, and more preferably 0.12 M to 0.24 M, as the final concentration of each. The final concentration of Tween may be adjusted to 0.01% to 0.2% by mass, preferably 0.02% to 0.15% by mass, and more preferably 0.03% to 0.12% by mass.

Furthermore, the temperature at the time of contact is preferably 45°C to 65°C and more preferably 50°C to 55°C, or is preferably 45°C to 70°C and more preferably 50°C to 65°C. The time for contacting is not limited as long as a hybridization reaction occurs so that mutation can be detected; however, a shorter time is preferred in order to suppress a non-specific reaction. For example, the contact time may be adjusted to 15 minutes to 4 hours, preferably 20 minutes to 3 hours, and more preferably 30 minutes to 2 hours.

### <Detection of nucleic acid (amplification product)>

The nucleic acid captured by the probes in the microarray is detected by the above-described step c).

The method for detection is not limited as long as the captured nucleic acid is detected, and any known method may be used. For example, a method of performing color development analysis or fluorescence intensity analysis using a fluorescent material or a luminescent material as a label substrate, or a method based on visual inspection, may be used.

More specifically, determination of the presence or absence and quantitative determination of the captured nucleic acid may be carried out with a fluoro-imaging analyzer or a CCD camera, etc. Quantitative determination of nucleic acid with higher reliability can be achieved by monitoring the amount of fluorescence over time using a quantitative real-time PCR analyzer that is being frequently used in recent years.

Furthermore, color development method may also be carried out using a color developing reagent that does or does not utilize an enzymatic reaction, or the like. Such a method may involve direct observation by visual inspection, or scanning with an optical scanner.

The method for detecting a nucleic acid of this invention may be applied to an analysis of 30 sites of mutation in the *β*-globin gene as disclosed in the Sequence Listing, but a detection kit for sites other than the mutation sites disclosed in this invention can also be produced by designing and using appropriate probes.

### (4) Kit

According to this invention, a microarray having a primer set and the probe group of this invention may also be used as a kit for detecting mutations in the *β*-globin gene. Regarding the primer set, a set of an oligonucleotide primer having the sequence set forth in SEQ ID NO:21 and an oligonucleotide primer having the sequence in SEQ ID NO:22; or a set of an oligonucleotide primer having the sequence set forth in SEQ ID NO:23 and an oligonucleotide primer having the sequence set forth in SEQ ID NO:24 may be more suitably used.

### 4. Method for evaluating microarray probe

As discussed previously, when detection of polymorphism is carried out using a microarray, it is preferred that the probe used does not cause non-specific hybridization. That is, a probe that does not cause non-specific hybridization is evaluated to have high performance.

Thus, this invention provides the following method for quantitatively evaluating the performance of a probe.

The method for evaluating a microarray probe includes the following steps:
1) a step of plotting the fluorescence coordinates obtained by hybridizing a control nucleic acid for the first polymorphism with a probe pair for polymorphism detection consisting of a probe for detecting the first polymorphism and a probe for detecting the second polymorphism, in a fluorescence coordinate system which includes a Y-axis representing the signal intensity obtainable when the probe for detecting the first polymorphism is hybridized, and an X-axis representing the signal intensity obtainable when the probe for detecting the second polymorphism is hybridized;
2) a step of defining a value which is inversely proportional to the slope of a straight line that passes through the intersection O between the Y-axis and the X-axis and the fluorescence coordinates plotted in the step 1), as a correction value C; and
3) a step of carrying out the steps 1) and 2) on plural probe pairs for polymorphism detection, comparing the correction values C between the various probes, and determining a probe pair having the minimum correction value C as probes appropriate for detecting the first polymorphism.

According to this invention, the first polymorphism and the second polymorphism are different alleles for the same polymorphism. That is, the first polymorphism is a first allele, and the second polymorphism is a second allele corresponding to the first allele.

Hereinafter, a summary of the various steps will be described.

### Step 1): Plotting step

First, in step 1), the signal intensities obtained when a control nucleic acid for the first polymorphism is hybridized to a probe pair for polymorphism detection consisting of a probe for detecting the first polymorphism and a probe for detecting the second polymorphism, are plotted in a fluorescence coordinate system. In regard to the fluorescence coordinate system of this invention, the Y-axis represents the signal intensity obtainable when the probe for detecting the first polymorphism is hybridized, and the X-axis represents the signal intensity obtainable when the probe for detecting the second polymorphism is hybridized. Here, the intersection between the Y-axis and the X-axis is designated as "O". Furthermore, the Y-axis and the X-axis may be orthogonal with each other, or may not be orthogonal with each other.

Through the above plotting process, fluorescence coordinates P(x₁,y₁) representing the fluorescence characteristics of the probe for detecting the first polymorphism are obtained.

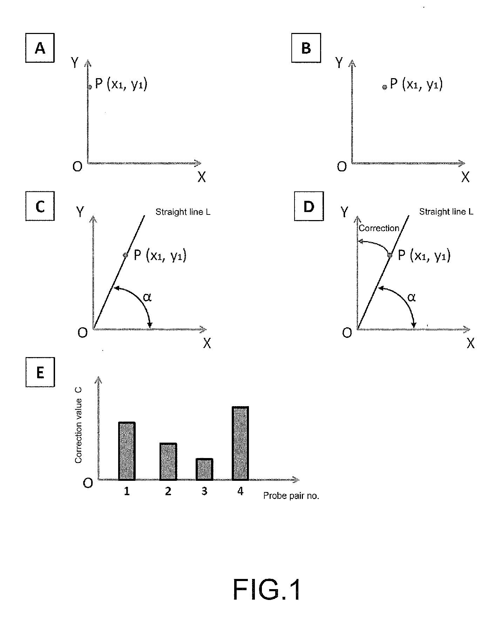

The fluorescence coordinates P of an ideal probe that does not cause non-specific hybridization are such that x₁ = 0 and y₁ > 0 (see FIG. 1: Panel A).

However, in reality, many probes cause non-specific hybridization to a certain extent. Therefore, the fluorescence coordinates P of many probes are such that x₁ > 0 and y₁ > 0 (see FIG. 1: Panel B).

Hybridization is made in a hybridization solution. A hybridization solution is a solution which enables a hybridization reaction between a control nucleic acid and a probe. More specifically, a hybridization solution is a solution obtained by mixing, with a buffer solution such as Tris/HCl buffer, a solution mixed with a salt, such as a NaCl solution or a MgCl₂ solution, or a SSC solution, and a surfactant such as SDS or Tween 20. Generally, when a reaction is carried out, it is preferred to use TNT buffer (mixed solution of a Tris/HCl buffer solution, a NaCl solution, and a Tween solution), in which crystals of a surfactant such as SDS are not precipitated at the time of cooling. The final concentration of the hybridization solution is preferably 0.06 M to 0.48 M, and more preferably 0.12 M to 0.24 M. Furthermore, the temperature at the time of contact is preferably 45°C to 70°C, and more preferably 50°C to 65°C. Regarding the contact time, a shorter contact time is more preferred, as long as a hybridization reaction occurs so that detection can be made. The contact time is usually 15 minutes to 4 hours, preferably 20 minutes to 3 hours, and more preferably 30 minutes to 2 hours.

The signal is a value obtained by digitizing the amount of control nucleic acids captured by the probes as a result of the above hybridization. In general, the signal may be obtained by causing a fluorescent substance or a luminescent substance to bind to the nucleic acid that is hybridized to a probe, and measuring the intensity of the fluorescence or developed color emitted from the probe region. Specifically, the signal may be obtained using a fluoroimaging analyzer, or a CCD camera, etc.

Signals "corresponding to" a probe may include signals originating from the background, while signals "originating from" a probe mean signals originating from the intrinsic specificity of the probe.

### Step 2): Determination of correction value

Next, a straight line L that passes through the fluorescence coordinates P thus plotted and the intersection O is determined, and a value that is inversely proportional to the slope of this straight line L may be designated as a correction value C (C > 0).

As a specific example, the correction value C may also be a value inversely proportional to the radian angle *α* (0≤*α*≤π/2) formed by the straight line L and the X-axis (see FIG. 1: Panel C). That is, when the fluorescence coordinates P are corrected to exist on the Y-axis, it is necessary to amplify the angle *α* to [(π/2)÷*α*] times (Fig. 1: Panel D), but the correction value C may also be defined as "C = (π/2)÷*α*" (C ≥ 1), based on this degree of amplification.

### Step 3): Comparison of probe pairs

Usually, in order to detect a single polymorphism, plural candidate probes are prepared. Therefore, it is necessary to carry out the above-described steps 1) and 2) on plural candidate probes and thereby determine the correction value C of each probe.

When a comparison is made between the correction values C obtained in this manner, the performance of the candidate probes may be compared and evaluated (see FIG. 1: Panel E).

As discussed above, in an ideal probe, since the fluorescence coordinates P exist on the Y-axis, the relationship *α* = π/2 is established. Therefore, in an ideal probe, the correction value C is equal to (π/2)÷(π/2) = 1.

On the other hand, in the case of a probe causing non-specific hybridization to a certain extent, since *α* < π/2, the correction value C is larger than 1. For example, in a case of *α* = π/4, the correction value C is equal to 2, and in a case of *α* = π/6, the correction value C is equal to 3.

Therefore, according to the method of this invention, it is considered that as the value of the correction value C is smaller, the probe has superior performance. That is, a probe having the minimum value of the correction value C (that is, the angle *α* is maximal) is determined as a probe appropriate for detecting the first polymorphism. For example, in the example of FIG. 1: Panel E, the probe pair no. 3 is determined as a probe appropriate for detecting the first polymorphism.

The processes described above may also be subjected to various modifications.

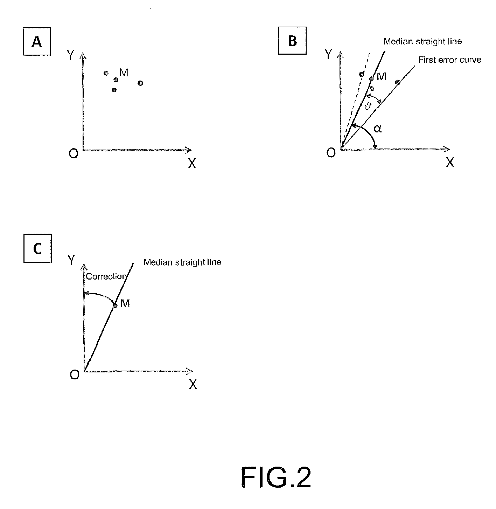

For example, in regard to step 1), two or more points of fluorescence coordinates may be obtained by repeating hybridization between a control nucleic acid and a probe two or more times (see FIG. 2: Panel A; in this case, three points of fluorescence coordinates). In this case, a representative value M of the two or more points of the fluorescence coordinates thus obtained is determined, and the straight line L according to step 2) may be a median straight line that passes through the intersection O and the representative value M (see FIG. 2: Panel B). Here, the representative value is a value representing plural values. Examples of the representative value include an average value, a median value, and a weighted average value, but from the viewpoint of robustness against outliers, a median value is preferred.

Furthermore, in regard to step 1), among the various straight lines (since there are two or more points of fluorescence coordinates, there are also two or more straight lines) that each pass through the intersection O and the fluorescence coordinates, a straight line having a difference in the slope with the median straight line is selected, and this may be designated as an error straight line (when a straight line having the maximum difference in the slope is selected, this is designated as a first error straight line) (see FIG. 2: Panel B).

When a first error straight line is determined, step 2) includes:
a) a process of determining the angle *α* (radian) between the median straight line and the X-axis, and
   determining the correction value C = π/2 ÷ *α* (see FIG. 2: Panel C); and
b) a process of designating the angle formed by the median straight line and the error straight line (when a straight line having the maximum difference in the slope is selected, this is done with the first error straight line) as an error angle θ (radian), and
   defining a correction error angle θ' (radian) = θ (radian) x the correction value C.

The error angle may be subjected to constant multiplication described above as necessary. Also, a straight line having the largest difference as a straight line having a difference may be designated as the first error straight line, and a range of the error angle added with a confidence interval may be determined from the angle differences with plural straight lines having a difference.

On the other hand, steps 4) to 6) corresponding to the steps 1) to 3) may also be carried out for a second probe for polymorphism detection, using a control nucleic acid for the second polymorphism.

Specifically, steps 4) to 6) are as follows:
4) a step of plotting fluorescence coordinates obtained by hybridizing a control nucleic acid for the second polymorphism with a probe pair for polymorphism detection consisting of a probe for detecting the first polymorphism and a probe for detecting the second polymorphism;
5) a step of designating a value which is proportional to the slope of the straight line that passes through the intersection O and the fluorescence coordinates plotted in step 4), as a correction value C₂; and
6) a step of carrying out steps 4) and 5) on plural probe pairs for polymorphism detection, comparing the correction values C₂ between the respective probes, and determining a probe pair having the minimum correction value C₂ as a probe appropriate for detecting the second polymorphism.

### Step 4): Plotting step

In step 4), the signal intensities obtained when a control nucleic acid for the second polymorphism is hybridized to a probe pair for polymorphism detection consisting of a probe for detecting the first polymorphism and a probe for detecting the second polymorphism, are plotted in a fluorescence coordinate system.

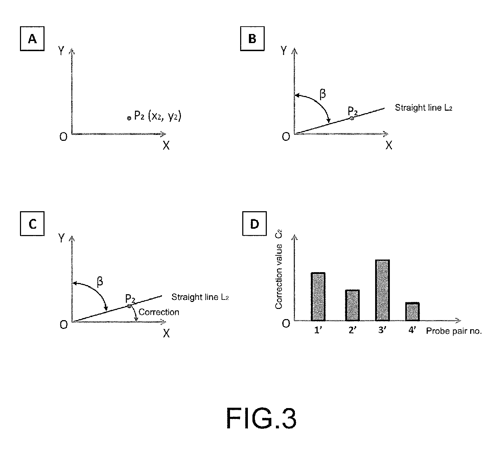

Through the above plotting process, fluorescence coordinates P₂(x₂,y₂) representing the fluorescence characteristics of the second probe for polymorphism detection are obtained (see FIG. 3: Panel A).

### Step 5): Determination of correction value

Next, a straight line L₂ that passes through the fluorescence coordinates P₂ thus plotted and the intersection O is determined, and a value that is proportional to the slope of this straight line L₂ may be designated as a correction value C₂ (C₂ > 0).

As a specific example, the correction value C₂ may be a value inversely proportional to the radian angle *β*(0 ≤ *β* ≤ π/2) formed by the straight line L₂ and the Y-axis (that is, proportional to the slope of L₂ (π/2*β*)) (see FIG. 3: Panel B). When the fluorescence coordinates P₂ are corrected to exist on the X-axis, it is necessary to amplify the angle *β* to [(π/2)÷*β*] times (see FIG. 3: Panel C), but the correction value C₂ may also be defined as "C₂ = (π/2)÷*β*" (C₂≥1), based on this degree of amplification.

### Step 6): Comparison of probe pairs

Similarly to the above step 3), it is necessary to determine the correction values C₂ of various probes by carrying out the above-described steps 4) and 5) on plural candidate probes.

When a comparison is made between the correction values C₂ obtained in this manner, the performance of the candidate probes may be compared and evaluated (see FIG. 3: Panel D).

As discussed above, in an ideal probe pair, since the fluorescence coordinates P₂ exist on the X-axis, the relationship "*β*=π/2" is established. Therefore, in an ideal probe, the correction value C₂ is equal to (π/2)÷(π/2)=1.

On the other hand, in the case of a probe which causes non-specific hybridization to a certain extent, since *β*<π/2, the correction value C₂ is larger than 1. For example, in a case of *β*=π/4, the correction value C₂ is equal to 2, and in a case of *β*=π/6, C₂ is equal to 3.

Therefore, it is considered that as the value of the correction value C₂ is smaller, the probe has superior performance. That is, a probe having the minimum value of the correction value C₂ is determined as a probe appropriate for detecting the second polymorphism. For example, in the example of FIG. 3: Panel D, the probe pair no. 4' is determined as a probe appropriate for detecting the second polymorphism.

The processes described above may also be subjected to various modifications.

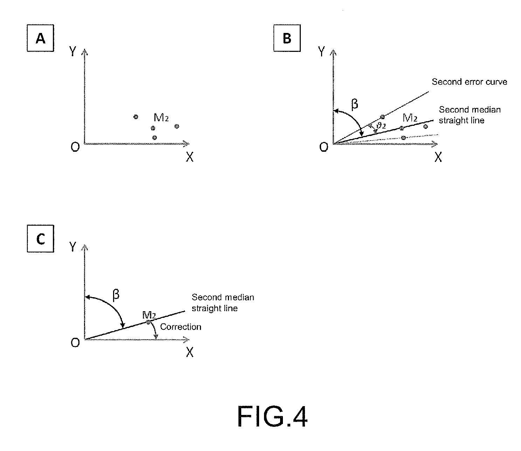

For example, in regard to step (4), two or more points of fluorescence coordinates may be obtained by repeating hybridization between a control nucleic acid and a probe two or more times (see FIG. 4: Panel A; in this case, three points of fluorescence coordinates). In this case, a representative value M₂ of the two or more points of fluorescence coordinates thus obtained is determined, and the straight line L₂ according to step 2) may be a second median straight line that passes through the intersection O and the representative value M₂ (FIG. 4: Panel B).

Here, the representative value is a value representing plural values. Examples thereof include an average value, a median value, and a weighted average value, but from the viewpoint of robustness against outliers, a median value is preferred.

Furthermore, in regard to step 4), among the various straight lines (since there are two or more points of fluorescence coordinates, there are also two or more straight lines) that each pass through the intersection O and the fluorescence coordinates, a straight line having a difference in the slope from the second median straight line is selected, and this may be designated as an error straight line (when a straight line having the maximum difference in the slope is selected, this is designated as a second error straight line) (see FIG. 4: Panel B).

When a second error straight line is determined, step 2) includes:
a) a process of determining the angle *β* (radian) between the second median straight line and the Y-axis, and
   determining the correction value C₂ = π/2÷β; and
b) a process of designating the angle formed by the second median straight line and the error straight line (when a straight line having the maximum difference in the slope is selected, this is done with the second error straight line) as an error angle θ₂ (radian), and
   defining a correction error angle θ₂' (radian) = θ₂ (radian) x the correction value C₂.

The error angle may be subjected to constant multiplication described above as necessary. Also, a straight line having the largest difference as a straight line having a difference may be designated as the second error straight line, and a range of the error angle added with a confidence interval may be determined from the angle differences with plural straight lines having a difference.

Furthermore, this invention provides a method of displaying the correction value C (or C₂) of the probe evaluated by the method described above, and the performance of the probe is evaluated. This invention also provides a method of displaying corrected coordinates and a corrected error range that have been corrected using the correction value C (or C₂).

In the evaluation method of this invention, the performance between various probes can be easily compared, and it is also possible to determine the genotype by considering the error range.

Hereinafter, this invention will be described more specifically by way of Examples, but these Examples are only for illustrative purposes and are not intended to limit this invention.

### EXAMPLES

### [Example 1]

An investigation was conducted on detecting the mutation at 25 sites in the *β*-globin gene all at once using a DNA microarray. The sites of mutation to be detected are presented in the following Table 1.

**Table 1: Mutation sites in β-globin**

| Mutation Site | HGVS nomenclature | | Mutation Site | HGVS nomenclature |
|---|---|---|---|---|
| 1 | c-137C>A | | 14 | c.92+1G>T |
| 2 | c-81A>G | | 15 | c.92+5G>C |
| 3 | c-80T>C | | 16 | c.108C>A |
| 4 | c-78A>G | | 17 | c.170G>A |
| 5 | c 2T>G | | 18 | c.216_217insA |
| 6 | c 5T>C | | 19 | c.251G>A |
| 7 | c 19G>A | | 20 | c.316-197C>T |
| 8 | c 27_28insG | | 21 | c.364G>C |
| 9 | c 46delT | | 22 | c.370_3777delACCCCACC |
| 10 | c 52A>T | | 23 | c.380T>G |
| 11 | c 59A>G | | 24 | c.410G>A |
| 12 | c 79G>A | | 25 | c.441_442insAC |
| 13 | c 84_85insC | | | |

Among these, for probes that detect mutation c.52A>T, c.84_85insC, c.364G>C and c.380T>G, since the difficulty in the probe design is high due to the characteristics of vicinal base sequences, the investigation was conducted first.

### 1. Production of through-hole type DNA microarray

A DNA microarray was produced as follows.

### <1-1. Preparation of probe>

Oligonucleotides having the sequences set forth in SEQ ID NO:1 to 18 that served as probes were synthesized.

These were synthesized as oligonucleotides each having an aminohexyl group introduced at the 5'-terminus thereof. After the synthesis, the oligonucleotide was caused to react with methacrylic anhydride, and the product was further purified and fractionated by HPLC. Thus, 5'-terminal vinylated oligonucleotides having the base sequences set forth in SEQ ID NOs:1 to 18 of Table 2 were obtained. Regarding the features of the sequences, SEQ ID NOs:1 and 2 are probes that are affected by the mutation of mutation c.59A>T adjacent to mutation c.52A>T, while SEQ ID NOs:3 and 4 have inosine introduced therein as a universal base that is hybridized to the mutation of mutation c.59A>T adjacent to mutation c.52A>T.

Similarly, SEQ ID NOs:5 and 6 are probes that are affected by the mutation of mutation c.79G>A adjacent to mutation c.84_85insC, while SEQ ID NOs:7 and 8 have inosine introduced therein as a universal base that is hybridized to the mutation of mutation c.79G>A adjacent to mutation c.84_85insC.

Furthermore, in SEQ ID NOs:11 and 12, a site of a different base for detecting mutation is located at the position six bases away from the 3-terminus of the probe, and SEQ ID NOs:17 and 18 have "AA" introduced at both termini.

**Table 2: Candidate probe sequences**

| Probe pair candidate 1 for detecting mutation c.52A>T | | |
|---|---|---|
| 12_1_c.52A>T | CTGTGGGGCAAGGTGAACG | SEQ ID NO:1 |
| 12_2_c.52A>T | CTGTGGGGCTAGGTGAACG | SEQ ID NO:2 |

| Probe pair candidate 2 for detecting mutation c.52A>T | | |
|---|---|---|
| 12_c.52A>T①kail | GGCAAGGTGAICGTGGATG | SEQ ID NO:3 |
| 12_c.52A>T②kail | GGCTAGGTGAICGTGGATG | SEQ ID NO:4 |
| | | |

| Probe pair candidate 1 for detecting mutation c.84_85insC | | |
|---|---|---|
| 15_1_c.84_85insC | TGGTGAGGCCCTGGGCAGG | SEQ ID NO:5 |
| 15_2_c.84_85insC | TGGTGAGGCCCCTGGGCAG | SEQ ID NO:6 |

| Probe pair candidate 2 for detecting mutation c.84_85insC | | |
|---|---|---|
| 15_c.84_85insC①kail | GTIAGGCCCTGGGCAG | SEQ ID NO:7 |
| 15_c.84_85insC②kail | TIAGGCCCCTGGGCAG | SEQ ID NO:8 |
| | | |

| Probe pair candidate 1 for detecting mutation c.364G>C | | |
|---|---|---|
| 26_1_c.364G>C | TTTGGCAAAGAATTCACCC | SEQ ID NO:9 |
| 26_2_c.364G>C | TTTGGCAAACAATTCACCC | SEQ ID NO:10 |

| Probe pair candidate 2 for detecting mutation c.364G>C | | |
|---|---|---|
| 26_c.364G>C①kail | CCATCACTTTGGCAAAGAATTC | SEQ ID NO:11 |
| 26_c.364G>C②kail | CCATCACTTTGGCAAACAATTC | SEQ ID NO:12 |
| | | |

| Probe pair candidate 1 for detecting mutation c.380T>G | | |
|---|---|---|
| 28_1_c.380T>G | ACCCCACCAGTGCAGGCTG | SEQ ID NO:13 |
| 28_2_c.380T>G | ACCCCACCAGGGCAGGCTG | SEQ ID NO:14 |

| Probe pair candidate 2 for detecting mutation c.380T>G | | |
|---|---|---|
| 28_1_c.380T>G_20111104 | CAGTGCAGGCTGCCTATCAGA | SEQ ID NO:15 |
| 28_2_c.380T>G_20111104 | CAGGGCAGGCTGCCTATCAGA | SEQ ID NO:16 |

| Probe pair candidate 3 for detecting mutation c.380T>G | | |
|---|---|---|
| 27 28 probe ① (Wt-T) | AACCCACCAGTGCAGGCAA | SEQ ID NO:17 |
| 27 28 probe ② (Wt-G) | AACCCACCAGGGCAGGCAA | SEQ ID NO:18 |

The oligonucleotides having the sequences set forth in SEQ ID NOs:1 to 18 may be hybridized to portions of the human *β*-globin gene sequences.

### <1-2. DNA microarray>

In this Example, nucleic acid microarrays (Genopal™, by Mitsubishi Rayon Co., Ltd.), which used the probes described in Table 1 (SEQ ID NOs:1 to 18), and used water instead of the nucleic acid probes for those sites not mounted with probes, were used.

### 2. Evaluation of probes for mutation detection in β-globin gene

### <2-1. Production of plasmid template DNA>

The *β*-globin gene sequence is encoded on the complementary strand sequence from the 5246730^{th} base to the 5248465^{th} base of NCBI Reference Sequence: NC_000011.9 (sequence length: 135006516 bases). The sequence is set forth in SEQ ID NO:19. Further, the exon region in the genomic DNA sequence was characterized by comparing with the sequence of NM_000518.4|Homo sapiens hemoglobin, beta (HBB), mRNA set forth in SEQ ID NO:20 (protein coding region: 51^{st} base to 494^{th} base; exon 1: 1^{st} base to 142^{nd} base; exon 2: 143^{rd} base to 365^{th} base; exon 3: 366^{th} base to 626^{th} base).

In SEQ ID NO:19, the sequence sites described in italicized characters represents the positions of the primer sequences of SEQ ID NOs:21 to 24, the underlined sequences represent exon regions, and the regions surrounded by rectangles represent UTR regions.

A wild type template for the *β*-globin gene was prepared by synthesizing a plasmid containing the sequence set forth in SEQ ID NO:19 (inserted into a pUC57 vector using the artificial gene synthesis service provided by BEX Co., Ltd.), and the template was prepared into a solution having a concentration of 10 ng/l.

Furthermore, similarly to this, individual plasmid DNAs (25 kinds), which have mutations introduced at the positions of the notation of mutation according to the HGVS nomenclature as shown in Table 1, were produced, and those were also prepared into solutions having a concentration of 10 ng/l.

### Primer pair <SEQ ID NOs:21, 22, 23 and 24>

| | | |
|---|---|---|
| SEQ ID NO:21 | Amplicon1F | ACTCCTAAGCCAGTGCCAGA |
| SEQ ID NO:22 | Amplicon1R | cy5-CACTCAGTGTGGCAAAGGTG |
| SEQ ID NO:23 | MRC-Amplicon2F | GTATCATGCCTCTTTGCACCATTC |
| SEQ ID NO:24 | MRC-Amplicon2R | cy5-CAGATGCTCAAGGCCCTTCATA |

<SEQ ID NO:19>
>gi|224589802:c5248465-5246730 homo sapiens chromosome 11 GRCh37.5
Primary Assembly 
AACTCCTAAGCCAGTGCCAGAAGAGCCAAGGACAGGTACGGCTGTCATCACTTAGACCTCACCCTGTGG
AGCCACACCCTAGGGTTGGCCAATCTACTCCCAGGAGCAGGGAGGGCAGGAGCCAGGGCTGGGCATAAA
AGTCAGGGCAGAGCCATCTATTGCTTACATTTGCTTCTGACACAACTGTGTTCACTAGCAACCTCAAA
CAGACACCATGGTGCATCTGACTCCTGAGGAGAAGTCTGCCGTTACTGCCCTGTGGGGCAAGGTGAAC
CTGGATGAAGTTGGTGGTGAGGCCCTGGGCAGGTTGGTATCAAGGTTACAAGACAGGTTTAAGGAGACC
AATAGAAACTGGGCATGTGGAGACAGAGAAGACTCTTGGGTTTCTGATAGGCACTGACTCTCTCTGCCT
ATTGGTCTATTTTCCCACCCTTAGGCTGCTGGTGGTCTACCCTTGGACCCAGAGGTTCTTTGAGTCCTT
TGGGGATCTGTCCACTCCTGATGCTGTTATGGGCAACCCTAAGGTGAAGGCTCATGGCAAGAAAGTGCT
CGGTGCCTTTAGTGATGGCCTGGCTCACCTGGACAACCTCAAGGGCACCTTTGCCACACTGAGTGAGCT
GCACTGTGACAAGCTGCACGTGGATCCTGAGAACTTCAGGGTGAGTCTATGGGACGCTTGATGTTTTCT
TTCCCCTTCTTTTCTATGGTTAAGTTCATGTCATAGGAAGGGGATAAGTAACAGGGTACAGTTTAGAAT
GGGAAACAGACGAATGATTGCATCAGTGTGGAAGTCTCAGGATCGTTTTAGTTTCTTTTATTTGCTGTT
CATAACAATTGTTTTCTTTTGTTTAATTCTTGCTTTCTTTTTTTTTCTTCTCCGCAATTTTTACTATTA
TACTTAATGCCTTAACATTGTGTATAACAAAAGGAAATATCTCTGAGATACATTAAGTAACTTAAAAAA
AAACTTTACACAGTCTGCCTAGTACATTACTATTTGGAATATATGTGTGCTTATTTGCATATTCATAAT
CTCCCTACTTTATTTTCTTTTATTTTTAATTGATACATAATCATTATACATATTTATGGGTTAAAGTGT
AATGTTTTAATATGTGTACACATATTGACCAAATCAGGGTAATTTTGCATTTGTAATTTTAAAAAATGC
TTTCTTCTTTTAATATACTTTTTTGTTTATCTTATTTCTAATACTTTCCCTAATCTCTTTCTTTCAGGG
CAATAATGATACAATGTATCATGCCTCTTTGCACCATTCTAAAGAATAACAGTGATAATTTCTGGGTTA
AGGCAATAGCAATATCTCTGCATATAAATATTTCTGCATATAAATTGTAACTGATGTAAGAGGTTTCAT
ATTGCTAATAGCAGCTACAATCCAGCTACCATTCTGCTTTTATTTTATGGTTGGGATAAGGCTGGATTA
TTCTGAGTCCAAGCTAGGCCCTTTTGCTAATCATGTTCATACCTCTTATCTTCCTCCCACAGCTCCTGG
GCAACGTGCTGGTCTGTGTGCTGGCCCATCACTTTGGCAAAGAATTCACCCCACCAGTGCAGGCTGCCT
ATCAGAAAGTGGTGGCTGGTGTGGCTAATGCCCTGGCCCACAAGTATCACTAAGCTCGCTTTCTTGCT
GTCCAATTTCTATTAAAGGTTCCTTTGTTCCCTAAGTCCAACTACTAAACTGGGGGATATTATGAAGG
GCCTTGAGCATCGG
 <SEQ ID NO:20>

ACATTTGCTTCTGACACAACTGTGTTCACTAGCAACCTCAAACAGACACCATGGTGCATCTGACTCCTG
AGGAGAAGTCTGCCGTTACTGCCCTGTGGGGCAAGGTGAACGTGGATGAAGTTGGTGGTGAGGCCCTGG
GCAGGCTGCTGGTGGTCTACCCTTGGACCCAGAGGTTCTTTGAGTCCTTTGGGGATCTGTCCACTCCTG
ATGCTGTTATGGGCAACCCTAAGGTGAAGGCTCATGGCAAGAAAGTGCTCGGTGCCTTTAGTGATGGCC
TGGCTCACCTGGACAACCTCAAGGGCACCTTTGCCACACTGAGTGAGCTGCACTGTGACAAGCTGCACG
TGGATCCTGAGAACTTCAGGCTCCTGGGCAACGTGCTGGTCTGTGTGCTGGCCCATCACTTTGGCAAAG
AATTCACCCCACCAGTGCAGGCTGCCTATCAGAAAGTGGTGGCTGGTGTGGCTAATGCCCTGGCCCACA
AGTATCACTAAGCTCGCTTTCTTGCTGTCCAATTTCTATTAAAGGTTCCTTTGTTCCCTAAGTCCAACT
ACTAAACTGGGGGATATTATGAAGGGCCTTGAGCATCTGGATTCTGCCTAATAAAAAACATTTATTTTC
ATTGC

### <PCR reaction>

PCR reactions were carried out using five kinds of plasmid DNAs in total, namely, the wild type plasmid DNA, and the four kinds of mutant plasmid DNAs of Nos. 10, 13, 21 and 23 (including mutations c.52A>T, c.84_85insC, c.364G>C and c.380T>G) described in Table 1 as templates, and using two pairs of primers having the sequences of SEQ ID NOs:21 to 24. For the PCR reaction, a KOD FX Neo kit (Toyobo Co., Ltd.) was used.

### <PCR reaction liquid composition>

| | |
|---|---|
| Plasmid DNA solution (10 ng/µL) | 1 µL (wild type or mutant) |
| Amplicon1F primer (20 µM) | 1 µL |
| Amplicon1R primer (20 µM) | 1 µL |
| MRC-Amplicon2F primer (20 µM) | 0.5 µL |
| MRC-Amplicon2R primer (20 µM) | 0.5 µL |
| 2xbuffer | 50 µl |
| 2 mM dNTPs | 20 µl |
| MILLI Q water | 24 µl |
| KOD FX Neo | 2 µl |
| Reaction volume | 100 µl |

For the PCR reaction, a GeneAmp9700 thermal cycler was used, and the reaction was carried out in the Max mode. The temperature conditions are shown below.

### <PCR reaction temperature conditions>

| | |
|---|---|
| 95°C | for 10 minutes |
| (94°C for 30 seconds, 68°C for 30 seconds, and 72°C for 30 seconds) x 35 cycles | |
| 4°C | end of reaction |

The following buffer solution was added to 100 µl of the reaction liquid obtained after the reaction to obtain a final volume of 200 µl.

| | |
|---|---|
| Reaction liquid | 100 µl |
| 1M Tris/HCl (pH 7.5) buffer | 48 µl |
| 5M NaCl solution | 9.6 µl |
| 0.5% aqueous solution of Tween20 | 20 µl |
| MILLI Q water | 22.4 µl |
| Total | 200 µl |

Then, 200 µl of this solution was introduced into a chamber for exclusive use (described in http://www.mrc.co.jp/genome/about/usage.html), then the DNA microarrays were introduced therein, the chamber was covered with a lid, and the mixture was incubated at 55°C for 2 hours.

After the incubation, each of the chips was immersed in 10 ml of 0.24 M TNT buffer at 55°C for 20 min. Then, subsequently, each of the chips was immersed in 10 ml of 0.24 M TN buffer at 55° for 10 minutes to perform washing. After the washing, detection was performed.

The detection was carried out using an automated DNA microarray detection apparatus of a cooled CCD camera system. The DNA microarrays were subjected to image-capturing from the top of the wells for an exposure time of 4 seconds, and the fluorescent signals of Cy5 at the respective spots were detected. A spot where the probes on the microarrays were not mounted was designated as a blank spot, and the median value of the fluorescence intensity thereof was designated as a background value. Values obtained by subtracting the background value from the fluorescence intensities at all the spots were designated as signals of the respective probes.

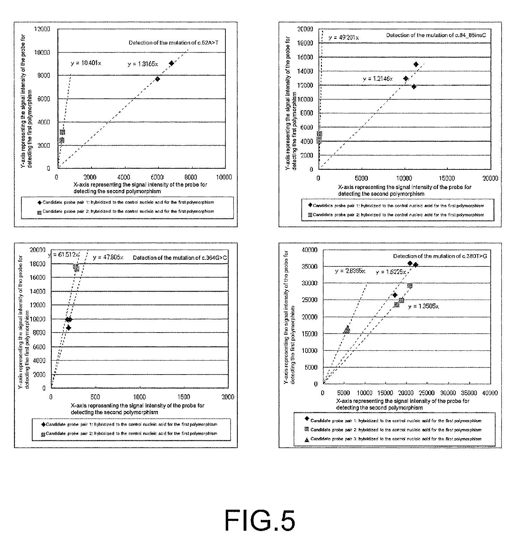

The results obtained by performing the experiment several times by employing the sequence of the wild type as a first polymorphism, the sequence of a mutant as a second polymorphism, and the control nucleic acid for first polymorphism as a wild type plasmid are presented in Table 3. Further, FIG. 5 shows the results of plotting the results of Table 3 in a fluorescence coordinate system which included a Y-axis representing the signal intensity obtainable when the probe for detecting the first polymorphism was hybridized, and an X-axis representing the signal intensity obtainable when the probe for detecting the second polymorphism was hybridized, with the X-axis and the Y-axis being orthogonal with each other (FIG. 5: a diagram obtained by plotting the results of performing hybridization of the first control nucleic acid several times in a fluorescence coordinate system representing the signal intensities of the probes for detecting the first and the second polymorphisms, and showing representative straight lines thereof).

A dotted line in FIG. 5 is a straight line (considered as a representative straight line) that links between the average signal intensity of the results of plural experiments (2 times or 3 times) for each candidate probe pair, and the zero point. A mathematical formula in the graph represents the formula for such straight lines.

Regarding the selection of the probe, a value that is inversely proportional to the slope of the representative straight line is designated as a correction value C, this is carried out for plural probe pairs for polymorphism detection to compare the correction values, and a probe pair having the minimum correction value C is selected. In the present investigation, the correction value C was calculated by the formula "π/2 ÷ (the angle (radian) formed by the representative straight line and the X-axis)." Among the respective probe pairs, the following probe pairs could be selected among the probe candidates as probe pairs having favorable performance:
the pair of SEQ ID NOs:3 and 4 being selected from the pair of SEQ ID NOs:1 and 2 and the pair of SEQ ID NOs:3 and 4, as a probe for detecting mutation of c.52A>T;
the pair of SEQ ID NOs:7 and 8 being selected from the pair of SEQ ID NOs:5 and 6 and the pair of SEQ ID NOs:7 and 8, as a probe for detecting mutation of c.84_85insC;
the pair of SEQ ID NOs:11 and 12 being selected from the pair of SEQ ID NOs:9 and 10 and the pair of SEQ ID NOs:11 and 12, as a probe for detecting mutation of c.364G>C; and
the pair of SEQ ID NOs:17 and 18 being selected from the pair of SEQ ID NOs:13 and 14, the pair of SEQ ID NOs:15 and 16, and the pair of SEQ ID NOs:17 and 18, as a probe for detecting mutation of c.380T>G.

**Table 3: Results obtained by performing the experiment plural times using a wild type plasmid (control nucleic acid for first polymorphism)**

| | | | | | Fluorescence obtained by hybridizing control nucleic acid for first polymorphism (wild type) with probe pair for polymorphism detection | | |
|---|---|---|---|---|---|---|---|
| Site to be detected | | | Probe name | Probe sequence | Signal intensity of 1^{st} test | Signal intensity of 2^{nd} test | Signal intensity of 3^{rd} test |
| c.52A>T | Pair of candidate 1 | Probe for first polymorphism detection | 12_1_c.52A>T | CTGTGGGGC**A**AGGTGAACG | 9034 | 7672 | |
| | | Probe for second polymorphism detection | 12_2_c.52A>T | CTGTGGGGC**T**AGGTGAACG | 6783 | 5918 | |
| | Pair of candidate 2 | Probe for first polymorphism detection | 12_c.52A>T①kail | GGC**A**AGGTGAICGTGGATG | 3106 | 2391 | |
| | | Probe for second polymorphism detection | 12_c.52A>T②kail | GGCTAGGTGAICGTGGATG | 289 | 242 | |
| | | | | | | | |

| Site to be detected | | | | | | | |
|---|---|---|---|---|---|---|---|
| c.84_85insC | Pair of candidate 1 | Probe for first polymorphism detection | 15_1_c.84_85insC | TGGTGAGGCCCTGGGCAGG | 14940 | 12913 | 11755 |
| | | Probe for second polymorphism detection | 15 2 c.84 85insC | TGGTGAGGCCCCTGGGCAG | 11353 | 10148 | 11091 |
| | Pair of candidate 2 | Probe for first polymorphism detection | 15_c.84_85insC①kail | GTIAGGCCCTGGGCAG | 5021 | 4024 | 4228 |
| | | Probe for second polymorphism detection | 15_c.84_85insC②kail | TIAGGCCCCTGGGCAG | 102 | 83 | 85 |
| | | | | | | | |

| Site to be detected | | | | | | | |
|---|---|---|---|---|---|---|---|
| c.364G>C | Pair of candidate 1 | Probe for first polymorphism detection | 26_1_c.364G>C | TTTGGCAAA**G**AATTCACCC | 9912 | 9880 | 8661 |
| | | Probe for second polymorphism detection | 26_2_c.364G>C | TTTGGCAAA**C**AATTCACCC | 214 | 183 | 196 |
| | Pair of candidate 2 | Probe for first polymorphism detection | 26_c.364G>C①kail | CCATCACTTTGGCAAA**G**AATTC | 17501 | 17171 | 17563 |
| | | Probe for second polymorphism detection | 26_c.364G>C②kail | CCATCACTTTGGCAAACAATTC | 285 | 286 | 278 |
| | | | | | | | |

| Site to be detected | | | | | | | |
|---|---|---|---|---|---|---|---|
| c.380T>G | Pair of candidate 1 | Probe for first polymorphism detection | 28_1_c.380T>G | ACCCCACCAG**T**GCAGGCTG | 35457 | 35876 | 26406 |
| | | Probe for second polymorphism detection | 28_2_c.380T>G | ACCCCACCAG**G**GCAGGCTG | 22184 | 20900 | 17312 |
| | Pair of candidate 2 | Probe for first polymorphism detection | 28_1_c.380T>G_20111104 | CAG**T**GCAGGCTGCCTATCAGA | 29308 | 24727 | 23549 |
| | | Probe for second polymorphism detection | 28_2_c.380T>G_20111104 | CAG**G**GCAGGCTGCCTATCAGA | 20927 | 18841 | 17785 |
| | Pair of candidate 3 | Probe for first polymorphism detection | 27_28 probe ① (Wt-T) | A**A**CCCACCAG**T**GCAGGCAA | 16676 | 16009 | 15860 |
| | | Probe for second polymorphism detection | 27_28 probe ② (Wt-T) | A**A**CCCACCAG**G**GCAGGC**AA** | 5830 | 5776 | 5505 |

In regard to the probe sequences presented in Table 3, a single-underlined base is the polymorphism to be detected, and a double-underlined base is a base that has been subjected to the modification of this invention (inosine substitution or adenine insertion).

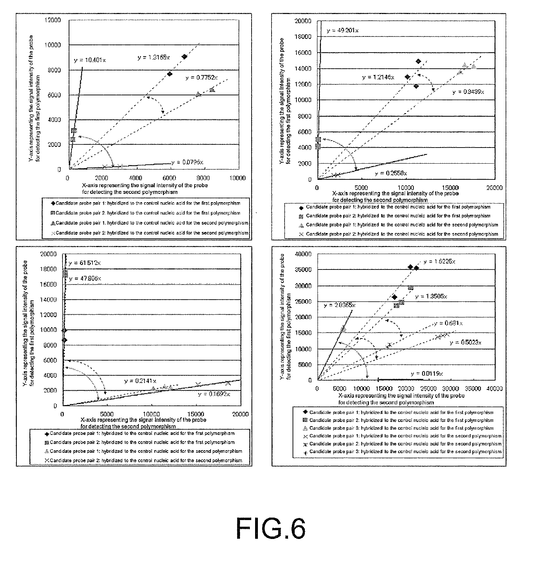

Similarly, the results obtained by performing the experiment several times by employing the sequence of the wild type as a first polymorphism, the sequence of a mutant as a second polymorphism, and the control nucleic acid for second polymorphism as a mutant plasmid are presented in Table 4. Further, FIG. 6 shows the results obtained by plotting the results of Tables 3 and 4 in a fluorescence coordinate system which included a Y-axis representing the signal intensity obtainable when the probe for detecting the first polymorphism was hybridized, and an X-axis representing the signal intensity obtainable when the probe for detecting the second polymorphism was hybridized, with the X-axis and the Y-axis being orthogonal with each other (in addition to FIG. 5, FIG. 6 is also a diagram obtained by plotting the results of performing hybridization of the second control nucleic acid several times, and showing representative straight lines thereof).

**Table 4: Results obtained by performing the experiment plural times using a mutant plasmid (control nucleic acid for second polymorphism)**

| | | | | | Fluorescence obtained by hybridizing the control nucleic acid for the first polymorphism (wild type) with a probe pair for polymorphism detection | | |
|---|---|---|---|---|---|---|---|
| Site to be detected | | | Probe name | Probe sequence | Signal intensity of 1^{st} test | Signal intensity of 2^{nd} test | Signal intensity of 3^{rd} test |
| c.52A>T | Pair of candidate 1 | Probe for detecting first polymorphism | 12_1_c.52A>T | CTGTGGGGC**A**AGGTGAACG | 9034 | 7672 | |
| | | Probe for detecting second polymorphism | 12_2_c.52A>T | CTGTGGGGC**T**AGGTGAACG | 6783 | 5918 | |
| | Pair of candidate 2 | Probe for detecting first polymorphism | 12_c.52A>T①kail | GGC**A**AGGTGA**I**CGTGGATG | 3106 | 2391 | |
| | | Probe for detecting second polymorphism | 12_c.52A>T②kail | GGC**T**AGGTGA**I**CGTGGATG | 289 | 242 | |
| | | | | | | | |

| Site to be detected | | | | | | | |
|---|---|---|---|---|---|---|---|
| c.84_85insC | Pair of candidate 1 | Probe for detecting first polymorphism | 15_1_c.84_85insC | TGGTGAGGCCCTGGGCAGG | 14940 | 12913 | 11755 |
| | | Probe for detecting second polymorphism | 15_2_c.84_85insC | TGGTGAGG**C**CCCTGGGCAG | 11353 | 10148 | 11091 |
| | Pair of candidate 2 | Probe for detecting first polymorphism | 15_c.84_85insC①kail | GT**I**AGGCCCTGGGCAG | 5021 | 4024 | 4228 |
| | | Probe for detecting second polymorphism | 15_c.84_85insC②kail | T**I**AGG**C**CCCTGGGCAG | 102 | 83 | 85 |
| | | | | | | | |

| Site to be detected | | | | | | | |
|---|---|---|---|---|---|---|---|
| c.364G>C | Pair of candidate 1 | Probe for detecting first polymorphism | 26_1_c.364G>C | TTTGGCAAA**G**AATTCACCC | 9912 | 9880 | 8661 |
| | | Probe for detecting second polymorphism | 26_2_c.364G>C | TTTGGCAAA**C**AATTCACCC | 214 | 183 | 196 |
| | Pair of candidate 2 | Probe for detecting first polymorphism | 26_c.364G>C①kail | CCATCACTTTGGCAAA**G**AATTC | 17501 | 17171 | 17563 |
| | | Probe for detecting second polymorphism | 26_c.364G>C②kail | CCATCACTTTGGCAAACAATTC | 285 | 286 | 278 |
| | | | | | | | |

| Site to be detected | | | | | | | |
|---|---|---|---|---|---|---|---|
| c.380T>G | Pair of candidate 1 | Probe for detecting first polymorphism | 28_1_c.380T>G | ACCCCACCAG**T**GCAGGCTG | 35457 | 35876 | 26406 |
| | | Probe for detecting second polymorphism | 28_2_c.380T>G | ACCCCACCAG**G**GCAGGCTG | 22184 | 20900 | 17312 |
| | Pair of candidate 2 | Probe for detecting first polymorphism | 28_1_c.380T>G_20111104 | CAG**T**GCAGGCTGCCTATCAGA | 29308 | 24727 | 23549 |
| | | Probe for detecting second polymorphism | 28_2_c.380T>G_20111104 | CAG**G**GCAGGCTGCCTATCAGA | 20927 | 18841 | 17785 |
| | Pair of candidate 3 | Probe for detecting first polymorphism | 27_28 probe ① (Wt-T) | A**A**CCCACCAG**T**GCAGGC**AA** | 16676 | 16009 | 15860 |
| | | Probe for detecting second polymorphism | 27_28 probe ② (Wt-G) | A**A**CCCACCAG**G**GCAGGC**AA** | 5830 | 5776 | 5505 |

In regard to the probe sequences indicated in Table 4, a single-underlined base is the polymorphism to be detected, and a double-underlined base is a base that has been subjected to the modification of this invention (inosine substitution or adenine insertion).

The series with "hybridized to the control nucleic acid for the second polymorphism" in the graph of FIG. 6 are the results obtained by hybridizing the mutant plasmid. A dotted line or a solid line is a representative straight line that links between the average signal intensity of the results of plural experiments (2 times or 3 times) for each candidate probe pair, and the zero point.

Regarding the selection of these probes, a value that is proportional to the slope of the representative straight line is designated as a correction value C₂, this is carried out for plural probe pairs for polymorphism detection to compare the correction values, and a probe pair having the minimum correction value C₂, which is appropriate for the detection of the second polymorphism (mutant), is selected.

In the present investigation, the correction value C₂ was calculated by the formula "π/2 ÷ (π/2 - angle (radian) formed by the representative straight line and the X-axis)." Among the respective probe pairs, the following probe pairs could be selected among the probe candidates as probe pairs having favorable performance:
the pair of SEQ ID NOs:3 and 4 being selected from the pair of SEQ ID NOs:1 and 2 and the pair of SEQ ID NOs:3 and 4, as a probe for detecting mutation of c.52A>T;
the pair of SEQ ID NOs:7 and 8 being selected from the pair of SEQ ID NOs:5 and 6 and the pair of SEQ ID NOs:7 and 8, as a probe for detecting mutation of c.84_85insC;
the pair of SEQ ID NOs:11 and 12 being selected from the pair of SEQ ID NOs:9 and 10 and the pair of SEQ ID NOs:11 and 12, as a probe for detecting mutation of c.364G>C; and
the pair of SEQ ID NOs:17 and 18 being selected from the pair of SEQ ID NOs:13 and 14, the pair of SEQ ID NOs:15 and 16, and the pair of SEQ ID NOs:17 and 18, as a probe for detecting mutation of c.380T>G.

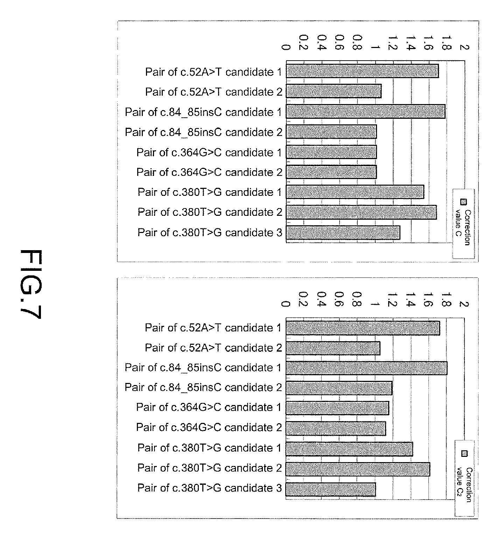

Graphs of the correction values C and C₂ described so far are presented in FIG. 7.

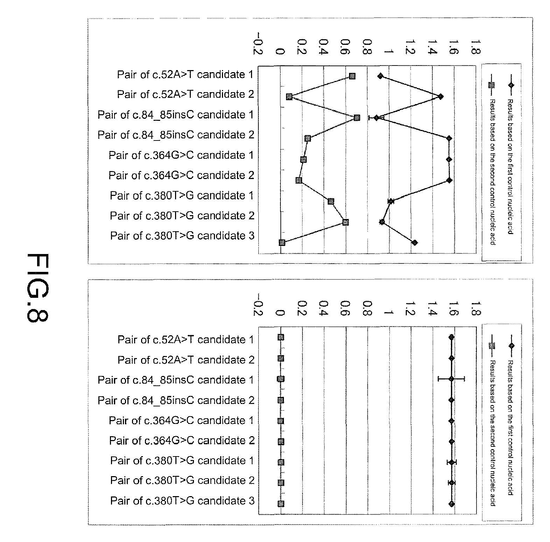

Subsequently to the evaluation of the probes, the error range that would be useful at the time of determining the genotype was set as shown in the following Table 5. The average value was calculated from the signal intensities obtained by repeating the procedure two or more times using the first control nucleic acid or the second control nucleic acid, and the average value was designated as the representative coordinates given by the probe pair. Further, the straight line passing through the representative coordinates and the zero point was designated as a representative straight line, the angle between the X-axis and the representative straight line was designated as a representative coordinate angle, and the angle (radian unit) between a straight line that linked the individual data and the zero point, and the representative straight line was calculated. The maximum angle was designated as an error angle. FIG. 8 shows the probe performance data obtained before and after the correction that was made using the correction values C and C₂, and the error angle.

**Table 5: Specific examples of the correction method of this invention**

| Site to be detected | Probe name | Probe name | Signal intensity of 1^{st} test | Signal intensity of 2^{nd} test | Signal intensity of 3^{rd} test | Angle of Test 1 | Angle of Test 2 | Angle of Test 3 | Representative coordinate | Angle of representative coordinate | Difference in angle from representative straight line | | | Angle of maximum difference | Correction value C | Error angle after correction |
|---|---|---|---|---|---|---|---|---|---|---|---|---|---|---|---|---|
| c.52A>T | Pair of candidate 1 | 12 1 c.52A>T | 9034 | 7672 | | 0.9268 | 0.9138 | | 8353 | 0.921 | 0.0060 | 0.0070 | | 0.0070 | 1.7060 | 0.0119 |
| | | 12_1_c.52A>T | 6783 | 5918 | | | | | 6351 | | | | | | | |
| | Pair of candidate 2 | 12_c.52A>T①kail | 3106 | 2391 | | 1.4781 | 1.4701 | | 2748 | 1.475 | 0.0035 | 0.045 | | 0.0045 | 1.0652 | 0.0048 |
| | | 12_c.52A>T②kail | 289 | 242 | | | | | 265 | | | | | | | |
| c.84_85insC | Pair of candidate 1 | 15 1 c.84 85insC | 14940 | 12913 | 11755 | 0.9210 | 0.9047 | 0.8145 | 13203 | 0.882 | 0.0387 | 0.0225 | 0.0678 | 0.0678 | 1.7804 | 0.1207 |
| | | 15_2_c.84_85insC | 11353 | 10148 | 11091 | | | | 10864 | | | | | | | |
| | Pair of candidate 2 | 15_c.84_85insC①kail | 5021 | 4024 | 4228 | 1.5505 | 1.5503 | 1.5506 | 4425 | 1.550 | 0.0001 | 0.0002 | 0.0001 | 0.0002 | 1.0131 | 0.0002 |
| | | 15_c.84_85insC②kail | 102 | 83 | 85 | | | | 90 | | | | | | | |
| c.364G>C | Pair of candidate 1 | 26 1 c.364G>C | 9912 | 9880 | 8661 | 1.5492 | 1.5523 | 1.5482 | 9484 | 1.550 | 0.0007 | 0.0023 | 0.0018 | 0.0023 | 1.0134 | 0.0023 |
| | | 26_2_c.364G>C | 214 | 183 | 196 | | | | 198 | | | | | | | |
| | Pair of candidate 2 | 26_c.364G>C①kail | 17501 | 17171 | 17563 | 1.5545 | 1.5542 | 1.5550 | 17412 | 1.555 | 0.0000 | 0.0004 | 0.0004 | 0.0004 | 1.0105 | 0.0004 |
| | | 26_c.364G>C②kail | 285 | 286 | 278 | | | | 283 | | | | | | | |
| c.380T>G | Pair of candidate 1 | 28 1 c.380T>G | 35457 | 35876 | 26406 | 1.0117 | 1.0433 | 0.9905 | 32580 | 1.017 | 0.0056 | 0.0260 | 0.0268 | 0.0268 | 1.5441 | 0.0414 |
| | | 28_2_c.380T>G | 22184 | 20900 | 17312 | | | | 20132 | | | | | | | |
| | Pair of candidate 2 | 28_1_c.380T>G_20111 104 | 29308 | 24727 | 23549 | 0.9507 | 0.9197 | 0.9240 | 25861 | 0.933 | 0.0182 | 0.0129 | 0.0086 | 0.0182 | 1.6844 | 0.0306 |
| | | 28_2_c.380T>G_20111 104 | 20927 | 18841 | 17785 | | | | 19184 | | | | | | | |
| | Pair of candidate 3 | 27 28 probe ① (Wt-T) | 16676 | 16009 | 15860 | 1.2345 | 1.2245 | 1.2367 | 16182 | 1.232 | 0.0026 | 0.0074 | 0.0048 | 0.0074 | 1.2751 | 0.0094 |
| | | 27_28 probe ② (Wt-G) | 5830 | 5776 | 5505 | | | | 5704 | | | | | | | |

### [Example 2]

In order to detect all at once the mutations at 25 sites in the *β*-globin gene using a DNA microarray, an array mounted with probes having the sequences set forth in SEQ ID NOs:3, 4, 7, 8, 11, 12, 17 and 18, and SEQ ID NOs:25 to 66 was produced.

The sites of mutation to be detected were the same as shown in Table 1 of Example 1, and the DNA microarray was also produced in the same manner as in Example 1.

### <PCR reaction>

PCR reactions were carried out using the mutant plasmid DNAs of Nos. 1 to 25 described in Table 1 as templates, and using two pairs of primers having the sequences of SEQ ID NOs:21 to 24. For the PCR reactions, an Ampdirect Plus kit (Shimadzu Corp.) was used.

### <PCR reaction liquid composition>

| | |
|---|---|
| Plasmid DNA solution (10 ng/µL) | 1 µL (wild type or mutant) |
| Amplicon1F primer (20 µM) | 1 µL |
| Amplicon1R primer (20 µM) | 1 µL |
| MRC-Amplicon2F primer (20 µM) | 0.5 µL |
| MRC-Amplicon2R primer (20 µM) | 0.5 µL |
| 2xAmpdirect buffer | 50 µl |
| BioTaq | 1 µl |
| (accompanying Ampdirect Plus kit) | |
| MILLI Q water | 45 µl |
| Total | 100 µl |

For the PCR reaction, a GeneAmp9700 thermal cycler was used, and the reaction was carried out in the Max mode. The temperature conditions are shown below.

### <PCR reaction temperature conditions>

| | |
|---|---|
| 95°C | for 10 minutes |
| (94°C for 30 seconds, 68°C for 30 seconds, and 72°C for 30 seconds) x 35 cycles | |
| 4°C | end of reaction |

The following buffer solution was added to 100 µl of the reaction liquid obtained after the reaction to obtain a final volume of 200 µl.

| | |
|---|---|
| Reaction liquid | 100 µl |
| 1M Tris/HCl (pH 7.5) buffer | 48 µl |
| 5M NaCl solution | 9.6 µl |
| 0.5% aqueous solution of Tween20 | 20 µl |
| MILLI Q water | 22.4 µl |
| Total | 200 µl |

Then, 200 µl of this solution was introduced into a chamber for exclusive use (described in http://www.mrc.co.jp/genome/about/usage.html), then the DNA microarrays were introduced therein, the chamber was covered with a lid, and the mixture was incubated at 55°C for 2 hours.

After the incubation, each of the chips was immersed in 10 ml of 0.24 M TNT buffer at 55°C for 20 min. Then, subsequently, each of the chips was immersed in 10 ml of 0.24 M TN buffer at 55° for 10 minutes to perform washing. After the washing, detection was performed.

The detection was carried out using an automated DNA microarray detection apparatus of a cooled CCD camera system. The DNA microarrays were subjected to image-capturing from the top of the wells for an exposure time of 4 seconds, and the fluorescent signals of Cy5 at the respective spots were detected. A spot where the probes on the microarrays were not mounted was designated as a blank spot, and the median value of the fluorescence intensity thereof was designated as the background value. Values obtained by subtracting the background value from the fluorescence intensities at all of the spots were designated as the signals of the respective probes.

The results are summarized in Table 6.

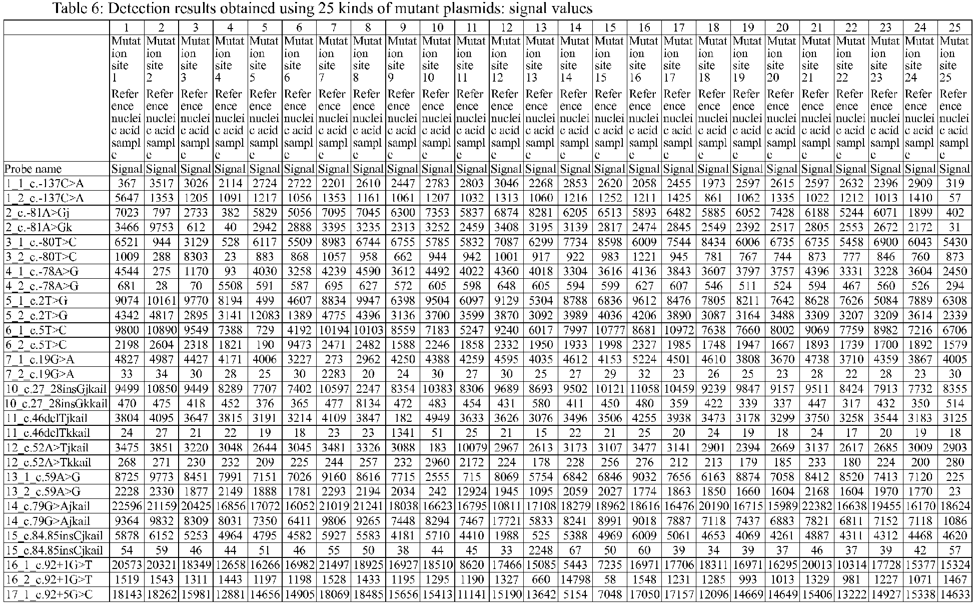

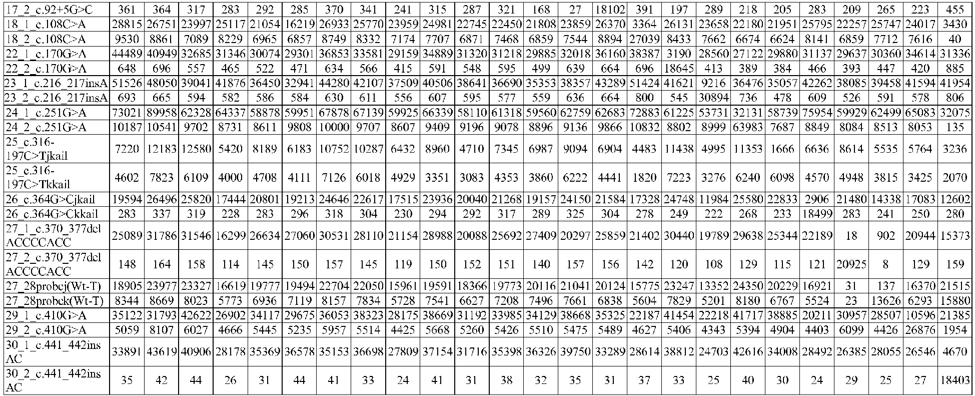

Subsequently, the ratio of signal originating from the wild type probe/signal originating from the mutant probe was calculated for each probe pair, and then the ratio was converted to radian unit (left side of Table 7). Then, among the data obtained by performing hybridization 25 times, the median value (radian) and the standard error (radian) of the Wild Type 24 data were calculated. The correction value C was computed by calculating the value of (π/2 ÷ the median value of the wild type), and the correction value C₂ was computed by calculating the value of (π/2 ÷ (π/2 - mutant)) (right side of Table 7).

Thereafter, regarding the error range, the standard error of the Wild Type 24 data was multiplied by the correction value C or the correction value C₂, and thereby the error range after correction was computed (right end of Table 7).

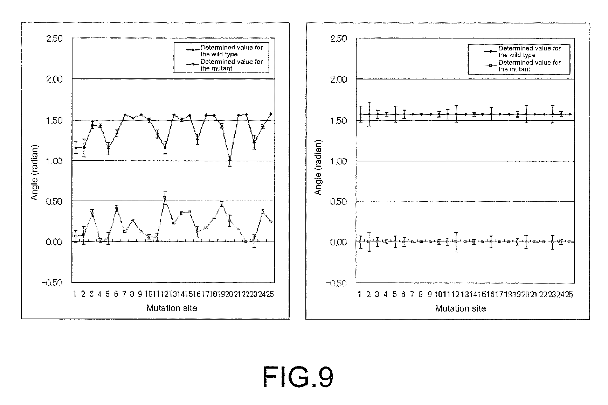

The data obtained before and after the correction using the upper limit or lower limit of the error range as the range of determination for the genotype are presented in FIG. 9 (FIG. 9: before correction and after correction of the data obtained from 25 kinds of plasmid-derived samples).

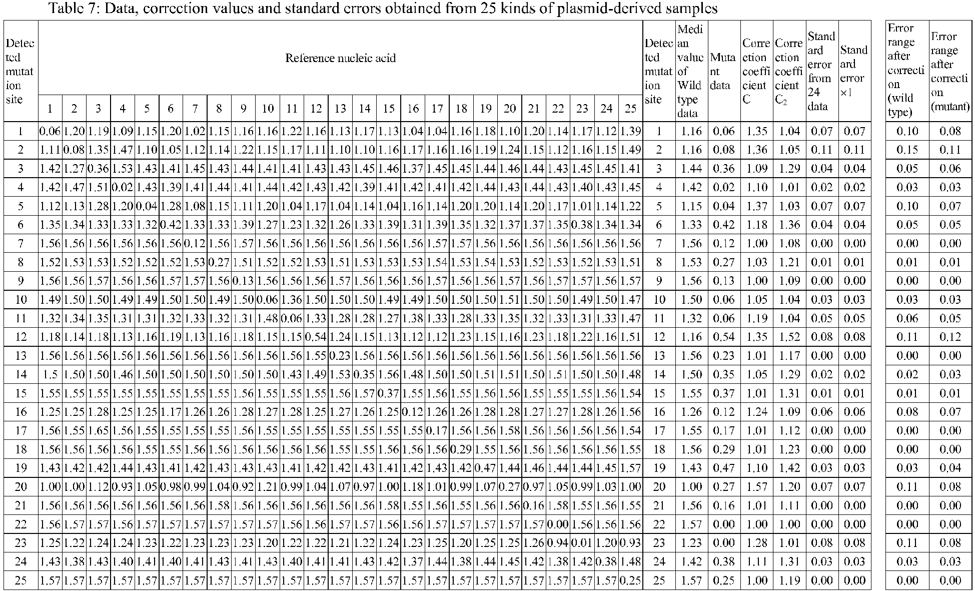

Apart from the present investigation, ECACC Ethnic Diversity DNA Panels (EDP-1) (Sigma Catalogue No: 07020701) were purchased, and one sample was subjected to an analysis of the base sequence using a sequencer. It was found that the sample was a sample having a heterozygous mutation at Mutation Site 12. Thus, subsequently, the data of the DNA chips were obtained by the same method as the method described in Example 2, using this sample, and the signal intensities shown in Table 8 were obtained.

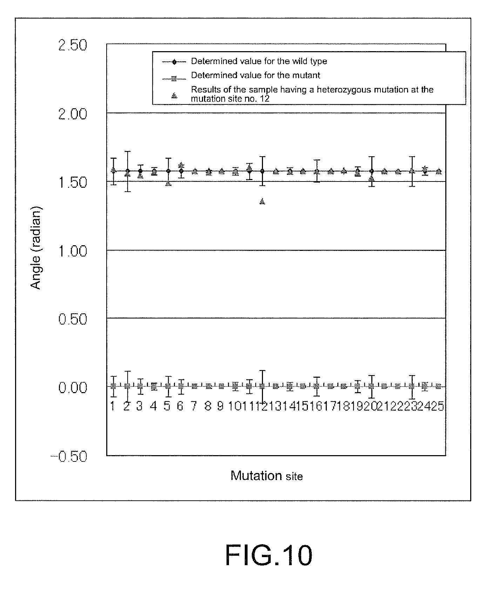

Subsequently, the signal intensities of Table 8 were used to calculate the ratio of (signal originating from wild type probe)/(signal originating from mutant probe) for each probe pair, and the resultant value was converted to radian unit and then multiplied by the correction value C in Table 7. The results are presented in Table 9. Furthermore, the data of Table 9 were superimposed on the graph for the data after correction of FIG. 9 (FIG. 10: results obtained by superimposing the data of Table 9 on the graph for the data after correction of FIG. 9), and only for Mutation Site No. 12, the data were plotted in the space indicated between the error bar of the wild type and the error bar of the mutant error bar. Thus, it could be determined that the mutation was heterozygous.

**Table 8: DNA chip signal values originating from a sample in which the mutation site 12 is heterozygous**

| Probe | Signal intensity |
|---|---|
| 1_1_c.-137C>A | 1909 |
| 1_2_c.-137C>A | 807 |
| 2_c.-81A>G① | 8805 |
| 2_c.-81A>G② | 3997 |
| 3_1_c.-80T>C | 7676 |
| 3_2_c.-80T>C | 1228 |
| 4_1_c.-78A>G | 5293 |
| 4_2_c.-78A>G | 799 |
| 5_1_c.2T>G | 9591 |
| 5_2_c.2T>G | 5016 |
| 6_1_c.5T>C | 14262 |
| 6_2_c.5T>C | 2827 |
| 7_1_71c.19G>A | 6619 |
| 7_2_c.19G>A | 38 |
| 10_c.27_28insG①kail | 13476 |
| 10_c.27_28insG②kail | 715 |
| 11_c.46delT①kail | 5258 |
| 11_c.46delT②kail | 28 |
| 12_c.52A>T①kail | 4699 |
| 12_c.52A>T②kail | 347 |
| 13_1_c.59A>G | 12209 |
| 13_2_c.59A>G | 2773 |
| 14_c.79G>A①kail | 22830 |
| 14_c.79G>A②kail | 14559 |
| 15_c.84_85insC①kail | 5048 |
| 15_c.84_85insC②kail | 51 |
| 16_1_c.92+1G>T | 27551 |
| 16_2_c.92+1G>T | 2101 |
| 17_1_c.92+5G>C | 23286 |
| 17_2_c.92+5G>C | 457 |
| 18_1_c.108C>A | 35463 |
| 18_2_c.108C>A | 11120 |
| 22_1_c.170G>A | 48518 |
| 22_2_c.170G>A | 703 |
| 23_1_c.216_217insA | 57316 |
| 23_2_c.216_217insA | 504 |
| 24_1_c.251G>A | 82857 |
| 24_2_c.251G>A | 12711 |
| 25_c.316-197C>T①kail | 4843 |
| 25_c.316-197C>T②kail | 3308 |
| 26_c.364G>C①kail | 22720 |
| 26_c.364G>C②kail | 304 |
| 27_1_c.370_377delACCCCACC | 30203 |
| 27_2_c.370_377delACCCCACC | 131 |
| 27_28 probe ① (Wt-T) | 22188 |
| 27_28 probe ② (Wt-G) | 7791 |
| 29_1_c.410G>A | 41279 |
| 29_2_c.410G>A | 5540 |
| 30_1_c.411_442insAC | 41848 |
| 30_2_c.411_442insAC | 41 |

**Table 9: Data obtained by correcting the data in Table 8**

| | Radian (before correction) | Correction coefficient C | Radian (after correction) |
|---|---|---|---|
| Detected mutation site 1 | 1.17 | 1.35 | 1.59 |
| Detected mutation site 2 | 1.14 | 1.36 | 1.56 |
| Detected mutation site 3 | 1.41 | 1.09 | 1.54 |
| Detected mutation site 4 | 1.42 | 1.10 | 1.57 |
| Detected mutation site 5 | 1.09 | 1.37 | 1.49 |
| Detected mutation site 6 | 1.38 | 1.18 | 1.62 |
| Detected mutation site 7 | 1.56 | 1.00 | 1.57 |
| Detected mutation site 8 | 1.52 | 1.03 | 1.56 |
| Detected mutation site 9 | 1.57 | 1.00 | 1.57 |
| Detected mutation site 10 | 1.50 | 1.05 | 1.57 |
| Detected mutation site 11 | 1.35 | 1.19 | 1.60 |
| Detected mutation site 12 | 1.00 | 1.35 | 1.36 |
| Detected mutation site 13 | 1.56 | 1.01 | 1.57 |
| Detected mutation site 14 | 1.49 | 1.05 | 1.56 |
| Detected mutation site 15 | 1.55 | 1.01 | 1.57 |
| Detected mutation site 16 | 1.27 | 1.24 | 1.58 |
| Detected mutation site 17 | 1.56 | 1.01 | 1.57 |
| Detected mutation site 18 | 1.56 | 1.01 | 1.58 |
| Detected mutation site 19 | 1.42 | 1.10 | 1.56 |
| Detected mutation site 20 | 0.97 | 1.57 | 1.52 |
| Detected mutation site 21 | 1.56 | 1.01 | 1.57 |
| Detected mutation site 22 | 1.57 | 1.00 | 1.57 |
| Detected mutation site 23 | 1.23 | 1.28 | 1.58 |
| Detected mutation site 24 | 1.44 | 1.11 | 1.59 |
| Detected mutation site 25 | 1.57 | 1.00 | 1.57 |

### Industrial Applicability

According to this invention, high-quality probes, a microarray having the same probes, and a method for evaluating the probes are provided.

### SEQUENCE LISTING FREE TEXT

SEQ ID NOs:1 to 18: Probes
SEQ ID NOs:21 to 24: Primers
SEQ ID NOs:25 to 66: Probes

SEQUENCE LISTING

## Claims

1. A probe for detecting a polynucleotide sequence having one or plural polymorphisms,
wherein the probe is hybridized to the sequence, and satisfies at least arbitrary one of the following requirements:
1) containing one or more non-complementary bases with respect to each end of the sequence;
2) a portion thereof corresponding to the polymorphisms not targeted for detection among the plural polymorphisms contained in the sequence contains universal bases; and
3) the polymorphism targeted for detection is located at a position six or less bases away from any one terminus of the probe.

2. A probe that is hybridized to a polynucleotide sequence having one or plural polymorphisms and being a sequence in which a sum of contents of guanine and cytosine is 63% or more, and satisfies the following requirements 1) and/or 2):
1) containing one or more non-complementary bases with respect to each end of the sequence; and
2) a portion thereof corresponding to the polymorphisms not targeted for detection among the plural polymorphisms contained in the sequence contains universal bases.

3. A probe that is hybridized to a polynucleotide sequence having one or plural polymorphisms and being a sequence in which a sum of contents of guanine and cytosine in the sequence is 45% or less,
wherein a polymorphism intended for detection is located at a position six or less bases away from any one terminus of the probe.

4. The probe according to any one of claims 1 to 3, wherein the polynucleotide sequence having one or plural polymorphisms is a human *β*-globin gene sequence.

5. The probe according to claim 2, wherein the sum of the contents of guanine and cytosine is 63% or more, and the polynucleotide sequence having one or plural polymorphisms comprises a sequence set forth in SEQ ID NO:3, 4, 7, 8, 17 or 18.

6. The probe according to claim 3, wherein the sum of the contents of guanine and cytosine is 45% or less, and the polynucleotide sequence having one or plural polymorphisms comprises a sequence set forth in SEQ ID NO:11 or 12.

7. A microarray comprising at least one of the sequences set forth in SEQ ID NOs:3, 4, 7, 8, 11, 12, 17 and 18.

8. A probe group for detecting mutations in a *β*-globin gene, comprising genes having the sequences set forth in SEQ ID NOs:3, 4, 7, 8, 11, 12, 17, 18 and 25 to 66.

9. A microarray comprising at least one of the sequences set forth in SEQ ID NOs:3, 4, 7, 8, 11, 12, 17, 18 and 25 to 66.

10. A *β*-thalassemia detection kit, comprising at least the probe according to any one of claims 1 to 9.

11. A *β*-thalassemia detection kit, comprising the microarray according to claim 7 or 9.

12. A kit for *β*-globin gene mutation detection, comprising:
a) i) an oligonucleotide primer having the sequence set forth in SEQ ID NO:21 and an oligonucleotide primer having the sequence set forth in SEQ ID NO:22, and/or ii) an oligonucleotide primer having the sequence set forth in SEQ ID NO:23 and an oligonucleotide primer having the sequence set forth in SEQ ID NO:24; and
b) the microarray according to claim 7 or 9.

13. A method for evaluating a microarray probe pair for polymorphism detection, comprising the following steps:
1) plotting fluorescence coordinates obtained by hybridizing a control nucleic acid for a first polymorphism with a probe pair for polymorphism detection, in a fluorescence coordinate system which includes a Y-axis representing the signal intensity obtainable when the probe for detecting the first polymorphism is hybridized, and an X-axis representing the signal intensity obtainable when the probe for detecting a second polymorphism is hybridized;
2) defining a value which is inversely proportional to a slope of a straight line that passes through an intersection O between the Y-axis and the X-axis and the fluorescence coordinates plotted in the step 1), as a correction value C; and
3) a step of carrying out the steps 1) and 2) on plural probe pairs for polymorphism detection, comparing the correction values C between the respective probes, and determining a probe pair having a minimum correction value C as probes appropriate for detecting the first polymorphism.

14. The method according to claim 13, wherein the Y-axis and the X-axis of the fluorescence coordinate system are orthogonal with each other.

15. The method according to claim 13 or 14, wherein
the step 1) involves obtaining two or more points of fluorescence coordinates by performing hybridization between the control nucleic acid and the probe two or more times, and determining a representative value M for the respective coordinates; and
in the step 2), the straight line is a median straight line that passes through the intersection O and the representative value M.

16. The method according to claim 14 or 15, wherein
the step 1) further involves a process of selecting a straight line having a difference in the slope from the median straight line among plural straight lines that pass through the intersection O and the two or more points of fluorescence coordinates, and designating the selected straight line as an error straight line; and
the step 2) includes:
a) a process of determining
the correction value C = π/2 ÷ *α*
from the angle *α* (radian) between the median straight line and the X-axis; and
b) a process of determining
a correction error angle θ' (radian) = θ (radian) x the correction value C
from an error angle θ (radian) which is an angle formed by the median straight line and the error straight line.

17. The method according to claims 13 to 16, further comprising the following steps:
4) plotting fluorescence coordinates obtained by hybridizing a control nucleic acid for the second polymorphism with a probe pair for detecting the second polymorphism;
5) defining a value which is proportional to a slope of a straight line that passes through the intersection O and the fluorescence coordinates plotted in the step 4), as a correction value C₂; and
6) performing steps 4) and 5) on plural probe pairs for detecting the second polymorphism, comparing the correction values C₂ between the respective probes, and determining a probe pair having a minimum correction value C₂ as a probe appropriate for detecting the second polymorphism.

18. The method according to claim 17, wherein
the step 4) involves obtaining two or more points of fluorescence coordinates by performing hybridization between the control nucleic acid for the second polymorphism and the probe for detecting the second polymorphism two or more times, and determining a representative value M₂ for the respective coordinates; and
in the step 5), the straight line is a second median straight line that passes through the intersection O and the representative value M₂.

19. The method according to claim 18, wherein
the step 4) further involves a process of selecting a straight line having a difference in the slope from the second median straight line among plural straight lines that pass through the intersection O and the two or more points of fluorescence coordinates, and designating the selected straight line as an error straight line; and
the step 5) includes:
c) a process of determining
the correction value C₂ = π/2 ÷ *β*
from the angle *β* (radian) between the second median straight line and the Y-axis; and
d) a process of determining
a second correction error angle θ₂' (radian) = θ₂ (radian) x the correction value C₂
from a second error angle θ₂ (radian) which is an angle formed by the second median straight line and the error straight line.

20. A genotype discrimination display program utilizing the method according to any one of claims 13 to 19.
